# EUROPEAN PATENT APPLICATION

(11) **EP 4 005 586 A1**
(43) Date of publication of application: **01.06.2022**
(21) Application number: 20844714.4
(22) Date of filing: 22.07.2020
(51) Int. Cl.: A61K 38/21, A61P 31/12, A61P 35/00

(54) **METHOD FOR TREATING DISEASES BASED ON INTERFERON**

(30) Priority: 22.07.2019 CN 201910660749
(71) Applicant: Xiamen Amoytop Biotech Co., Ltd., Xiamen, Fujian 361028 (CN); Biosteed Gene Transformation Tech.Co., Ltd., Xiamen, Fujian 361028 (CN)
(72) Inventor: SUN, Li, Xiamen, Fujian 361028 (CN); ZHOU, Weidong, Xiamen, Fujian 361028 (CN); LIAO, Xiaojin, Xiamen, Fujian 361028 (CN); ZHUANG, Lu, Xiamen, Fujian 361028 (CN); HE, Ruoyi, Xiamen, Fujian 361028 (CN); ZHOU, Ting, Xiamen, Fujian 361028 (CN); ZENG, Lingying, Xiamen, Fujian 361028 (CN); YANG, Meihua, Xiamen, Fujian 361028 (CN); WANG, Shiyuan, Xiamen, Fujian 361028 (CN); ZHENG, Jiehua, Xiamen, Fujian 361028 (CN); ZHANG, Linzhong, Xiamen, Fujian 361028 (CN)
(74) Representative: Vossius & Partner Patentanwälte Rechtsanwälte mbB
(86) International application number: PCT/CN2020/103613
(87) International publication number: WO 2021/013204

(57) **Abstract**

The invention relates to the field of biomedicine. In particular, the present invention relates to an interferon-based method for treating a disease, comprising intermittently administering an interferon-based therapeutic agent to a subject, where the disease is for example a viral infection or a cancer.

## Description

### Technical Field

The present invention relates to the field of biomedicine. In particular, the present invention relates to an interferon-based method for treating a disease, comprising intermittently administering an interferon-based therapeutic agent to a subject, where the disease is for example a viral infection or a cancer.

### Background

Interferons (IFN) are active proteins with multiple functions, and cytokines produced by monocytes and lymphocytes. With various biological activities, such as broad-spectrum anti-viral activity, influence on cell growth, differentiation, and regulation of immune functions, they are at present the most important anti-viral infection and anti-tumor biological products. As a broad-spectrum antiviral substance, interferon does not directly kill or inhibit a virus. Instead, it mainly acts on receptors on the surface of a cell, such that the cell may generate an antiviral protein, thereby inhibiting replication of viruses, while enhancing activity of natural killer cells (NK cells), macrophages and T lymphocytes, whereby the interferon provides immune regulation and enhances antiviral ability.

At present, interferons are categorized into Type I, Type II, and Type III interferons. The Type I interferons include IFN-α, IFN-β, and the like. The IFN-α interferons are mainly generated by monocytes-macrophages. In addition, B cells and fibroblasts may also synthesize the IFN-α interferons. The IFN-β interferons are mainly generated by fibroblasts. Both IFN-α and IFN-β interferons bind to the same receptors which are widely distributed in for example monocyte-macrophages, polymorphonuclear leukocytes, B cells, T cells, platelets, epithelial cells, endothelial cells and tumor cells. It is known that there are more than 23 subtypes of IFN α. There is only one subtype of IFN β. The Type II interferons, or γ interferons, are mainly generated by activated T cells (including Th0, Th1 cells and almost all CD8+ T cells) and NK cells, and belong to the so-called lymphokines. The IFN-γ may exist in the form of linking to an extracellular matrix, so it may control the cell growth by bystander effect. They may be distributed on the surface of almost all cells except mature red blood cells. There is only one subtype of IFN-γ. The Type III interferons mainly refer to the interferon λ.

Interferons, interferon mutants and interferon derivatives have been approved to be widely used in various treatments. Interferons and mutants that have been approved for human clinical treatment include interferon α 2a, interferon α 2b, interferon α 1b, compound interferon (Infergen), and interferon mutants (such as Novaferon), Interferon β, Interferon γ (1b), and the like. Interferon derivatives include Peginterferon α 2a, Peginterferon α 2b, integrated interferon and the like.

In addition, in recent years, with the discovery and elucidation of signal pathways such as TLRs, RLRs, and STING, a series of agonists that act on the above signal pathways to generate interferons have been discovered and elucidated, the use of which has also become a new direction for future interferon-based treatments. At present, this type of agonists has been used in the treatment of HBV and related tumors.

However, although certain therapeutic effects have been achieved with interferon substances and agonists thereof, application effects thereof are not so satisfactory. Taking the treatment of chronic hepatitis B as an example, it is shown that (see manual of Pegberon) only about 30% e-antigen seroconversion and only 1-3% of S-antigen clearance rate may be provided in e-antigen-positive chronic hepatitis B patients, whereby the clinical use effect is poor. A number of clinical studies have shown that with continuous use of interferon products, surface antigen indicators and e-antigen indicators in the early treatment process decline faster than those in the late stage, where changes in the late stage are slower. The results of studies on multiple genotypes of HBVs are similar, and it is shown in many studies that action mechanism thereof may be related to exhaustion of specific T cells and immunosuppression. It is progressing slowly on how to improve the treatment effect. The Type I IFN is the first batch of immunotherapy drugs approved by FDA for clinical therapy of cancers. It provided the best curative effect for hematological system tumors, followed by for lymphatic system tumors, and the worst curative effect for solid tumors, such as malignant melanoma, ovarian tumor, colorectal cancer, etc., where the curative effect is no more than 20%. In addition, the combined application of interferon with chemotherapy, radiotherapy and immunotherapy is at present a common anti-tumor therapy with interferons. For example, combined application of interferon with 5-fluorouracil for treating liver cancer and colorectal cancer, combined application of interferon with cisplatin, carboplatin and the like for treating lung cancers. Although these studies have achieved some results, the overall effect is not so satisfactory and the prognosis is poor. In addition, interferon is generally administered by continuous application of large doses in clinical practice, which inevitably causes problems such as drug-related toxicity, and poor compliance and tolerance of patients.

In summary, for application of interferon or combined application of interferon with anti-cancer or radiotherapy and other treatment methods, some progress has been made in the clinical application, but in general, such methods provide undesirable efficacy, poor prognosis, and large toxic side effects.

Therefore, it is still desirable in the art for improvement of interferon-based therapies to achieve better therapeutic effects.

### Brief Description of the Drawings

FIG. 1 shows the effect (surface antigen - time curve) of treating chronic hepatitis B through administering Pegasys or Pegberon by conventional regimen.
FIG. 2 shows that intermittent administration of interferon may improve the efficacy of interferon in later stages of treatment.
FIG. 3 shows that intermittent administration of interferon may repeatedly initiate immune clearance.
FIG. 4 shows that intermittent administration of interferon may make hepatitis B surface antigens in serum of a patient become negative.
FIG. 5 shows the SP Sepharose Fast Flow elution profile of mIFN-α4 fermentation supernatant.
FIG. 6 shows the non-reduced SDS-PAGE (14% separation gel, silver staining) electrophoresis results of SP Sepharose Fast Flow purified target mIFN-α4.
FIG. 7 shows the Q Sepharose Fast Flow purification elution profile of glycosyl-removed mIFN-α4.
FIG. 8 shows the non-reduced SDS-PAGE (14% separation gel, silver staining) electrophoresis results of Q Sepharose Fast Flow purified raw solution of glycosyl-removed mIFN-α4.
FIG. 9 shows the SP Sepharose Fast Flow purification elution profile of PEG-mIFN-α4.
FIG. 10 shows the non-reduced SDS-PAGE (silver staining) electrophoresis results of PEG-mIFN-α4 raw solution.
FIG. 11 shows the plasma concentration-time curve for a single subcutaneous injection of 1µg/mouse of PEG-mIFN-α4 to BALB/c mice.
FIG. 12 shows the survival curve of comparison experiment on the efficacy of continuous and intermittent administration of PEG-mIFN-α4 in the treatment of transplanted liver cancer H₂₂ in mice.
FIG. 13 shows the tumor changes of intermittent administration of interferon combined with administration of Gemcitabine in the treatment of liver cancer H₂₂ in mice.
FIG. 14 shows the tumor changes of intermittent administration of interferon combined with administration of Gemcitabine in the treatment of lung cancer LLC in mice.
FIG. 15 shows the tumor changes of intermittent administration of interferon combined with administration of Gemcitabine in the treatment of colorectal cancer CT26.
FIG. 16 shows the tumor changes of intermittent administration of interferon combined with administration of Gemcitabine in the treatment of melanoma B16.
FIG. 17 shows the survival curve of comparison experiment on the efficacy of intermittent administration of PEG-mIFN-α4 combined with administration of Gemcitabine in the treatment of transplanted liver cancer H22 in mice.
FIG. 18 shows the survival curve of intermittent administration of interferon combined with administration of anticancer agent Epirubicin in the treatment of transplanted liver cancer H22 in mice.
FIG. 19 shows the survival curve of intermittent administration of interferon combined with administration of anticancer agent Oxaliplatin in the treatment of transplanted liver cancer H22 in mice.
FIG. 20 shows the survival curve of intermittent administration of interferon combined with administration of anticancer agent Paclitaxel in the treatment of transplanted liver cancer H22 in mice.

### Detailed Description of the Invention

The inventors unexpectedly discovered that compared to continuous administration of interferon in a fixed course of treatment, significantly better therapeutic effects may be achieved through intermittent administration of interferon over multiple courses of treatment, for example, significantly better treatment effects with substantially same doses, with substantially same times of administration, or over a substantially same treatment period.

Without being limited by any theory, it is believed that continuous administration of interferon for a long time may cause the consumption of immune cells which are difficult to recover. The efficacy of interferon depends on the immune system. Therefore, an interferon-based therapy may achieve good therapeutic effects such as high virus clearance or tumor suppression in the early stage of treatment when immune cells are still sufficient. However, in the later stage of treatment, due to depletion of immune cells caused by long-term administration of the interferon, the therapeutic effect may be substantially reduced, and cannot be improved even if the interferon is continued to be administered. This problem may be avoided by intermittent administration of interferon. For example, good therapeutic efficacy may still be achieved if after administering an interferon for a period to achieve a certain therapeutic efficacy, administration of the interferon is suspended before partial immune suppression and exhaustion of immune cells for a period to allow the immune cells to recover as soon as possible, and then the administration of the interferon is resumed.

Therefore, in one aspect, the present invention provides a method for treating a disease in a subject, including intermittently administering an interferon-based therapeutic agent to the subject for a plurality of consecutive treatment courses.

The "interferon" may be a human interferon, for example, a Type I, II or III interferon, such as interferon α, interferon β, interferon γ or interferon λ, preferably interferon α.

As used herein, the "interferon-based therapeutic agent" refers to a therapeutic agent capable of generating at least part of the effects of a natural interferon.

For example, the "interferon-based therapeutic agents" may include a nature isolated or a recombinantly generated interferon, such as a Type I interferon, preferably an interferon α. A suitable interferon α includes but is not limited to interferon α 2a, interferon α 2b or interferon α 1b. The "interferon-based therapeutic agent" may also include an interferon mutant, such as Infergen (a recombinant integrated interferon).

The "interferon-based therapeutic agent" may also include an interferon derivative, such as PEGylated interferon or a mutant thereof, an albuminated interferon or a mutant thereof, and another protein and organic-modified interferon and a mutant thereof, and the like. Examples of the PEGylated modified interferon or the mutant thereof include, but are not limited to, peginterferon α 2a (e.g. Pegasys, 40Kd bi-branched UPEG-NHS modified), peginterferon α 2b (e.g., Pegintron, 12Kd linear PEG-SC modified), peginterferon α 2b (e.g., Pegberon, Y-type 40Kd PEG modified), cultured interferon α-2 (e.g., PEGINFER), Peginterferon λ, P1101, and the like.

In some embodiments, the "interferon-based therapeutic agent" includes a long-acting interferon, such as a PEGylated interferon or a mutant thereof. In some embodiments, the "interferon-based therapeutic agent" includes a short-acting interferon.

In some embodiments, the "interferon-based therapeutic agent" includes multiple types of interferons or mutants or derivatives thereof.

The term "interferon-based therapeutic agent" covers various therapeutic agents comprising interferons or mutants or derivatives thereof that have been approved for marketing. In some embodiments, the "interferon-based therapeutic agent" is Pegberon (Y-type 40Kd PEG modified, peginterferon α 2b, Amoytop). In some embodiments, the "interferon-based therapeutic agent" is Pegasys (peginterferon α 2a, Roche). In some embodiments, the "interferon-based therapeutic agent" is Pegintron (peginterferon α 2b injection, Schering-Plough). In some embodiments, the "interferon-based therapeutic agent" is Infergen (recombinant integrated interferon, Amgen, USA). In some embodiments, the "interferon-based therapeutic agent" is Intron A (recombinant human interferon α 2b, Schering-Plough). In some embodiments, the "interferon-based therapeutic agent" is Roferon-A (Interferon α 2a, Roche). In some embodiments, the "interferon-based therapeutic agent" is Hapgen (recombinant human interferon α 1b, Beijing Sanyuan Jiyin Engineering Co., Ltd.) In some embodiments, the "interferon-based therapeutic agent" is PEGINFER (PEGlated-integrated interferon α-2 injection, Beijing Kawin Technology Co., Ltd.). In some embodiments, the "interferon-based therapeutic agent" is peginterferon λ (Nanogen Pharmaceutical biotechbology). In some preferred embodiments, the interferon-based therapeutic agent includes interferon α 2b. In some preferred embodiments, the interferon-based therapeutic agent includes polyethylene glycol modified interferon α 2b. In some preferred embodiments, the interferon-based therapeutic agent is Pegberon.

In some embodiments, the "interferon-based therapeutic agent" includes an interferon or a mutant or derivative thereof, where the interferon or the mutant or derivative thereof includes an amino acid sequence of any one of SEQ ID NOs: 1-5, or the interferon or the mutant or derivative thereof includes an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to any one of SEQ ID NOs: 1-5.

In some embodiments, the "interferon-based therapeutic agent" also includes a nucleic acid molecule encoding an interferon or a mutant or derivative thereof, such as a recombinant nucleic acid expression vector. Suitable expression vectors, especially those suitable for therapeutic applications, may be easily determined by those skilled in the art.

The "interferon-based therapeutic agent" may also include a substance capable of promoting generation of endogenous interferons, such as agonists of the TLRs, RLRs, and STINGs signaling pathways. Examples of substances capable of promoting the generation of endogenous interferons include but are not limited to GS-9620, GS-9688, RO7020531, RO6864018, TQ-A3334, JNJ-4964, SB9200, MIW815, DMXAA, MK-1454, diABZI, an d the like^{[1]-[34]}.

As used herein, the term " consecutive treatment course" means that during the course of treatment, the administration of the therapeutic agent allows to continuously maintain the in vivo effective concentration of (exogenous or endogenous) interferon (for example, the effective blood concentration) within a patient, or to continuously maintain the in vivo concentration (for example, blood concentration) of neopterin (NPT), a main pharmacodynamic marker of interferons, within a patient to be higher than the concentration (initial concentration or baseline concentration) when the therapeutic agent is not administered. Since there is a good correlation between neopterin, a main pharmacodynamic marker of interferons, and the administration of interferons, it is particularly preferable to use the change in the in vivo concentration of neopterin (such as blood concentration) as the indicator of a "consecutive course of treatment". For example, a "consecutive course of treatment" may be defined as a course of treatment during which the administration of an interferon-based therapeutic agent for one or more times may keep the concentration of neopterin in a subject during substantially the entire course of treatment higher than the concentration of neopterin before the first administration (baseline concentration), for example approximately 110%, approximately 120%, approximately 130%, approximately 140%, approximately 150%, approximately 200%, approximately 250% or higher of the neopterin concentration before the first administration. The in vivo concentration of neopterin may be determined by methods known in the art.

In the consecutive treatment course, the administration scheme of the interferon-based therapeutic agent is generally determined by the characteristics of the selected therapeutic agent, such as its half-life in vivo. For example, in the consecutive treatment course, an long-acting interferon (with an in vivo half-life of generally 30-120 hours) may be administered about once a week, about once every two weeks, or may be administered once in a month or even a longer period in the case of increased dosage; and a short-acting interferon (with an in vivo half-life of generally 2-5 hours) may be administered once a day or once every two days or for three times a week, or may be administered once a week in the case of an increased dosage (for example, 9-36 MIU or higher), or may be administered for multiple times in a day, in the case of a reduced dosage. In the consecutive treatment course, the number of administrations of the interferon-based therapeutic agent is not particularly limited, as long as the above definition of the "consecutive treatment course" is met. Those skilled in the art may determine the consecutive treatment course based on the in vivo concentration (such as the blood concentration) of a pharmacodynamic marker such as neopterin of the interferon-based therapeutic agent.

In some embodiments, in the "consecutive treatment course", the "interferon-based therapeutic agent" may be administered in its conventional administration scheme. For example, Infergen (recombinant integrated interferon), interferon α 2b (such as Intron A), interferon α 2a (such as Roferon-A), interferon α 1b (such as Hapgen) may be administered once a day or once every two days or for three times a week in the dosage range of 3-18 MIU. Peginterferon α 2a (e.g., Pegasys), peginterferon α 2b (e.g. Pegintron or Pegberon), PEGlated-integrated interferon α-2 (such as PEGINFER) or peginterferon λ may be administered once a week in the dosage range of 45-270 µg. P1101 may be administered in about 400 µg once every two weeks. The albuminated interferon α 2b may be administered at about 900 to about 1800 µg once every two weeks, or about 1200 µg once every 4 weeks.

The duration of each one of the plurality of consecutive courses should allow the therapeutic agent to achieve a certain therapeutic effect, but should avoid excessive consumption of immune cells. The consumption of immune cells in the treatment course is usually identified by changes in treatment indicators. For example, when the relevant treatment indicator shows the worsened efficacy of the therapeutic agent, it may indicate excessive consumption of immune cells. For example, in the standard scheme for treating chronic hepatitis B with interferon (continuous treatment with interferon agents for 12 months), the reduction in surface antigens in the early stage of treatment (for example, the first 3 months or the first 6 months) is significantly greater than that in the late stage of treatment (for example, the late 9 months or the late 6 months), which shows partial immune suppression and depletion of immune cells (for example, after 3 months or 6 months of continuous treatment) (refer to Example 1).

In some embodiments, duration of each of the plurality of consecutive treatment courses is at least about 1 week.

In some embodiments, duration of each of the plurality of consecutive treatment courses is up to about 24 weeks.

In some embodiments, the duration of each of the plurality of consecutive treatment courses is about I week to about 24 weeks, for example, about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, or about 24 weeks.

In some preferred embodiments, the duration of each of the plurality of consecutive treatment courses is about 1 week to about 12 weeks. In some further preferred embodiments, the duration of each of the plurality of consecutive treatment courses is about 1 week to about 8 weeks. In some further preferred embodiments, the duration of each of the plurality of consecutive treatment courses is about 2 weeks to about 6 weeks. In some further preferred embodiments, the duration of each of the plurality of consecutive treatment courses is about 2 weeks.

In some embodiments, the end point of each consecutive treatment course (that is, the starting point of the interval between consecutive courses of treatment) may be the time of the last administration of the interferon-based therapeutic agent in the consecutive treatment course plus about 5 in vivo half-lives of the therapeutic agent. That is, the duration of the consecutive treatment course is the time period from the first administration to the last administration, plus about 5 in vivo half-lives of the therapeutic agent. It is believed that after 5 half-lives, the therapeutic agent will no longer generate a substantial therapeutic effect.

In the method of the present invention, the interval between the plurality of consecutive treatment courses may depend on the regeneration cycle of immune cells. The duration of the interval should allow the immune cells in the patient that have been reduced due to the treatment to be restored to a level that can effectively implement the treatment. It is generally believed that it takes about 1-2 weeks for the immune cells to proliferate. Therefore, the shortest interval between the plurality of consecutive treatment courses can be about 1 week.

In some embodiments, the interval between the plurality of consecutive treatment courses is at least about 1 week apart.

In some embodiments, the interval between the plurality of consecutive treatment courses is up to about 24 weeks.

In some embodiments, the interval between the plurality of consecutive treatment courses is about 1 week to about 24 weeks, for example, about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, or about 24 weeks.

In some preferred embodiments, the interval between the plurality of consecutive treatment courses is about 1 week to about 12 weeks. In some more preferred embodiments, the interval between the plurality of consecutive treatment courses is are about 1 week to about 8 weeks. In some further preferred embodiments, the interval between the plurality of consecutive treatment courses is about 2 weeks to about 6 weeks.

In some embodiments, the duration of each of the consecutive treatment courses is about 1 week to about 24 weeks, and the interval between the treatment courses is about 1 week to about 24 weeks. In some embodiments, the duration of each of the consecutive treatment courses is about 1 week to about 12 weeks, and the interval between the treatment courses is about 1 week to about 12 weeks. In some embodiments, the duration of each of the consecutive treatment courses is about 1 week to about 8 weeks, and the interval between the treatment courses is about 1 week to about 8 weeks. In some embodiments, the duration of each of the consecutive treatment courses is about 2 week to about 6 weeks, and the interval between the treatment courses is about 2 week to about 6 weeks. In some embodiments, the duration of each of the consecutive treatment courses is about 2 weeks, and the interval between the treatment courses is about 2 weeks.

In some embodiments, the "interferon-based therapeutic agent" is administered for at least 2, at least 3, at least 4, at least 5, at least 6, at least 7, at least 8, at least 9, at least 10, at least 11, at least 12, at least 13, at least 14, at least 15, at least 20, at least 25 or more consecutive treatment courses.

In some embodiments, the durations of the plurality of consecutive treatment courses are substantially the same.

In some embodiments, the time intervals between the treatment courses are substantially the same. In some specific embodiments, the interferon-based therapeutic agent is Pegberon, and the duration of each of the consecutive treatment courses is about 5 weeks to about 24 weeks, and the interval between the consecutive treatment courses is about 2 weeks to 8 weeks.

In some embodiments, the disease is a viral infection. The viral infection includes, but is not limited to, hepatitis B virus infection, hepatitis C virus infection, hepatitis D virus infection, HIV infection, EBV infection, HPV infection, CMV infection, herpes virus infection, and the like, preferably hepatitis B virus infection.

In some embodiments, the disease is a cancer. The cancer includes, but is not limited to, leukemia (such as acute lymphocytic leukemia (ALL), acute myeloid leukemia (AML), chronic myeloid leukemia (CML), chronic lymphocytic leukemia, polycapillary leukemia), liver cancer, lung cancer, colorectal cancer, skin cancer, stomach cancer, breast cancer, prostate cancer, non-Hodgkin's lymphoma, melanoma, multiple myeloma, laryngeal papilloma, follicular lymphoma, AIDS-related Kaposi's sarcoma, renal cell carcinoma, and the like. In some embodiments, the disease is myeloproliferative tumor (MPN). In some preferred embodiments, the disease is liver cancer, lung cancer, breast cancer, colorectal cancer or melanoma.

In some embodiments, the "interferon-based therapeutic agent" is administered in combination with an additional agent. The additional agent includes, but is not limited to, an antiviral agent, an anticancer agent, and the like.

In some embodiments, the antiviral agent is a penetration and uncoating inhibitor: including but not limited to amantadine, rimantadine, enfuvirtide, maraviro; a DNA polymerase inhibitor: including but not limited to acyclovir, ganciclovir, valacyclovir, famciclovir, foscarnet; a reverse transcriptase inhibitor: nucleosides: including but not limited to lamivudine, zidovudine, emtricitabine, Tenofovir, adefovir dipivoxil, TAF, entecavir, non-nucleosides: including but not limited to efavirenz, nevirapine; a protein inhibitor: saquinavir; neuraminidase inhibitors: including but not limited to Oseltamivir, zanamivir; a broad-spectrum antiviral drug: including but not limited to ribavirin, and the like In some preferred embodiments, the antiviral agent is entecavir, tenofovir, TAF and the like. In some embodiments, in the anti-HIV virus treatment, the additional agent includes, but is not limited to, emtricitabine, tenofovir, lamivudine, saquinavir, indinavir, ritonavir, nelfinavir, amprenavir, lopinavir, atazanavir, Kaletra, nevirapine, delavirdine, efavirenz, and the like.

In some embodiments, the interferon-based therapeutic agent is Pegberon and the antiviral agent is entecavir.

In some specific embodiments, the method is used to treat the hepatitis B virus infection; the interferon-based therapeutic agent is Pegberon; the duration of each of the consecutive treatment courses is about 5 weeks to about 24 weeks, and the interval between the consecutive treatment courses is about 2 weeks to 8 weeks; and the antiviral agent is entecavir, which is administered daily. In some embodiments, the anticancer agent is a chemotherapeutic agent, including but not limited to, such as an alkylating agent: Nimustine, Carmustine, Lomustine, Cyclophosphamide, Ifosfamide, glyciphosphoramide, semustine; such as a chemotherapeutic antimetabolite: deoxyfluoguanosine, doxifluguanidine, 5-fluorouracil, mercaptopurine, thioguanine, cytarabine, fluguanosine, tegafur, Gemcitabine, carmofur, hydroxyurea, methotrexate, UFT, Ancitabine, capecitabine; such as a chemotherapeutic anti-tumor antibiotic: actinomycin D, doxorubicin, daunorubicin, Epirubicin, mitomycin, pelomycin, pingyangmycin, pirarubicin; such as a chemotherapeutic anti-tumor animal and plant ingredient: irinotecan, harringtonine, hydroxycamptothecin, Vinorelbine, paclitaxel, albumin paclitaxel, taxotere, topotecan, vincristine, vindesine, Vindesine, vinblastine, teniposide, etoposide, elemene; such as anti-tumor drug hormones: Atamestane, Anastrozole, Aminoglutethimide, Letrozole, Formestane, Metasterone, Tamoxifen; such as a chemotherapeutic miscellaneous agent: asparaginase, carboplatin, cisplatin, Dacarbazine, Oxaliplatin, Loxadine, Eloxatin, Mitoxantrone, or Procarbazine.

In some embodiments, the additional drug is a small molecule targeting drug, including but not limited to imatinib, gefitinib, bortezomib, erlotinib, sorafenib, lenalidomide, Sunitinib, dasatinib, nilotinib, lapatinib, pazopanib, everolimus, vandetanib, crizotinib, verofinib, ruxolitinib, axitinib, vismodegib, carfilzomib, regorafenib, bosutinib, tofacitinib, carbotinib, panatinib, pomalidomide, trametinib, dabrafenib, Afatinib, Icotinib, Ibrutinib, Ceritinib, Idelaris, Apatinib, Pabuccilib, Levatinib, Axitinib, Icotinib , Apatinib, sonidegib, cobimetinib, osimertinib, alectinib, ixazomib.

In some embodiments, the additional agent is a tumor-associated antigen-specific antibody such as Rituxan, Herceptin and the like.

The additional agent may also be an immune checkpoint inhibitor, such as an inhibitor of PD1, PDL1, CTLA4, etc., such as a specific antibody. Examples of an immune checkpoint inhibitor include, but are not limited to, Nivolumab, Pembrolizumab, Atezolizumab, Durvalumab, Avelumab and the like.

In some specific embodiments, the chemotherapeutic agent is oxaliplatin. In some specific embodiments, the chemotherapeutic agent is epirubicin. In some specific embodiments, the chemotherapeutic agent is paclitaxel.

In some preferred embodiments, the chemotherapeutic agent is an antimetabolitic chemotherapeutic agent, such as gemcitabine, capecitabine, and ancitabine. In some most preferred embodiments, the chemotherapeutic agent is gemcitabine.

In some embodiments, the administration of the "interferon-based therapeutic agent" does not overlap with the administration of the additional agent.

In some embodiments, the additional agent is administered between the plurality of consecutive treatment courses. For example, the additional agent may be administered for a period of time during the administration interval of the "interferon-based therapeutic agent", said period of time shorter than or equal to the interval.

In some embodiments, the administration of the "interferon-based therapeutic agent" overlaps with the administration of the additional agent.

In some embodiments, the additional agent is administered during and between the plurality of consecutive treatment courses, that is, the additional agent is administered during the entire treatment period (completely overlapped).

The administration of the additional agent such as an antiviral agent during the entire treatment period or within the treatment interval of the "interferon-based therapeutic agent" is particularly preferred for the treatment of a viral infection such as hepatitis B virus infection, because reinfection of the virus during the period in which the administration of the "interferon-based therapeutic agent" is suspended may be controlled or avoided. In some embodiments, the additional agent is administered according to a conventional scheme, for example, its conventional administration scheme for the disease being treated.

In some embodiments, the method of the invention results in a reduction of viral load in the subject, for example, a reduction of at least 10%, at least 20%, at least 30%, at least 40%, at least 50%, at least 60%, at least 70%, at least 80%, at least 90%, at least 95%, or at least 99%. Preferably, the method of the present invention results in substantial elimination of the viruses in the subject, for example, undetectable by conventional methods. Preferably, the method of the invention results in the alleviation or elimination of one or more symptoms associated with the viral infection.

In some embodiments, the method of the invention results in tumor regression or reduction in tumor volume or prolonged survival of the subject. In particular, the method of the present invention results in: reduction of the number of cancer cells, reduction of tumor volume, inhibition (i.e., slow-down or stop) of the infiltration of cancer cells into peripheral organs, inhibition (i.e., slow-down or stop) of tumor metastasis, inhibition of tumor growth, and/or alleviation of one or more symptoms associated with the cancer.

In one aspect, the present invention provides a pharmaceutical composition including an interferon-based therapeutic agent for use in the treatment of a disease in a subject by a method of the present invention. In some embodiments, the pharmaceutical composition also includes an additional agent. The interferon-based therapeutic agent and the chemotherapeutic agent are as defined above.

In one aspect, the present invention provides the use of an interferon-based therapeutic agent in preparation of a pharmaceutical composition for treating a disease in a subject by a method of the present invention. In some embodiments, the pharmaceutical composition also includes an additional agent. The interferon-based therapeutic agent and the chemotherapeutic agent are as defined above.

In one aspect, the present invention provides the use of an interferon-based therapeutic agent in preparation of a pharmaceutical composition for treating a disease in a subject in combination with an additional agent. The interferon-based therapeutic agent and the chemotherapeutic agent are as defined above. The interferon-based therapeutic agent and/or the additional agent may be administered according to the method of the present invention.

In one aspect, the present invention provides the use of an interferon-based therapeutic agent in preparation of a pharmaceutical composition for enhancing the efficacy of an additional agent. The interferon-based therapeutic agent and the chemotherapeutic agent are as defined above. The interferon-based therapeutic agent and/or the additional agent may be administered according to the method of the present invention.

In one aspect, the present invention provides a kit including an interferon-based therapeutic agent and instructions for use, where the instructions for use provide a description that the interferon-based therapeutic agent may be according to the method of the present invention or be administered according to the method of the present invention to treat a disease in a subject. In some embodiments, the kit also includes an additional agent. The interferon-based therapeutic agent and the chemotherapeutic agent are as defined above.

### Examples

The following examples are only provided for better explaining the present invention and are not intended to limit the present invention in any way.

### Example 1. Conventional interferon-based hepatitis B therapy

The inventors collected hepatitis B surface e antigen data of hepatitis B patients who were treated with peginterferon α 2a (Pegasys) and peginterferon α 2b (Y type 40kD Pegberon). These patients were treated continuously according to the treatment scheme stated in the drug instructions. The treatment scheme of Pegasys: continuous treatment was performed with solely Pegasys in a dosage of 180 micrograms per week for 48 weeks. The treatment scheme of Pegberon: continuous treatment was performed with solely Pegberon in a dosage of 180 micrograms per week for 48 weeks.

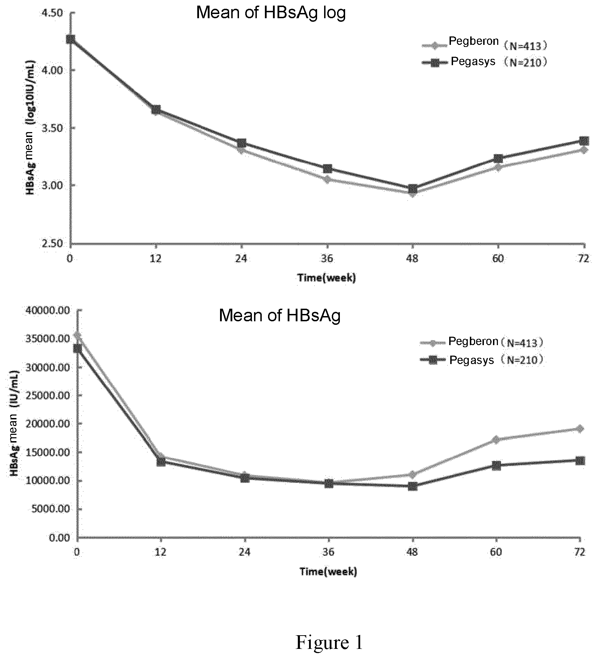

The results are shown in FIG. 1. In the treatment of chronic hepatitis B with Peginterferon α 2a (Pegasys) and peginterferon α 2b (Y-type 40kD Pegberon), the decline of surface antigens was in line with characteristics of faster decline in the early stage and slower decline in the later stage. It is shown in the average data graph of the declines in e antigen-positive chronic hepatitis B surface antigens in the control treatment with Pegberon and Pegasys, the treatment effect was better in the first 24 weeks than in the last 24 weeks, and the treatment effect was better in the first 12 weeks than in the last 36 weeks. Continuous use of interferon for more than 12 weeks or more than 24 weeks may cause partial immune suppression and exhaustion of immune cells, which may lead to poor follow-up treatment effects.

### Example 2. Improved interferon-based therapy

As shown in Example 1, continuous interferon treatment provides poor efficacy. The inventors surprisingly found that by intermittently administering interferon, a significantly better therapeutic effect may be obtained.

In a patient with chronic hepatitis B, after using Pegberon for 48 weeks, administration of the Pegberon was suspended and the nucleotide analogue Entecavir (ETV) was routinely administered to inhibit viral replication. After administration of Pegberon was suspended for 5 weeks, Pegberon was used again for 8 weeks, then suspended again for 6 weeks. The process was repeated in the next stage.

Administration of Pegberon: 180 micrograms per week. The nucleotide analogue ETV was administrated orally in 0.5 mg per day.

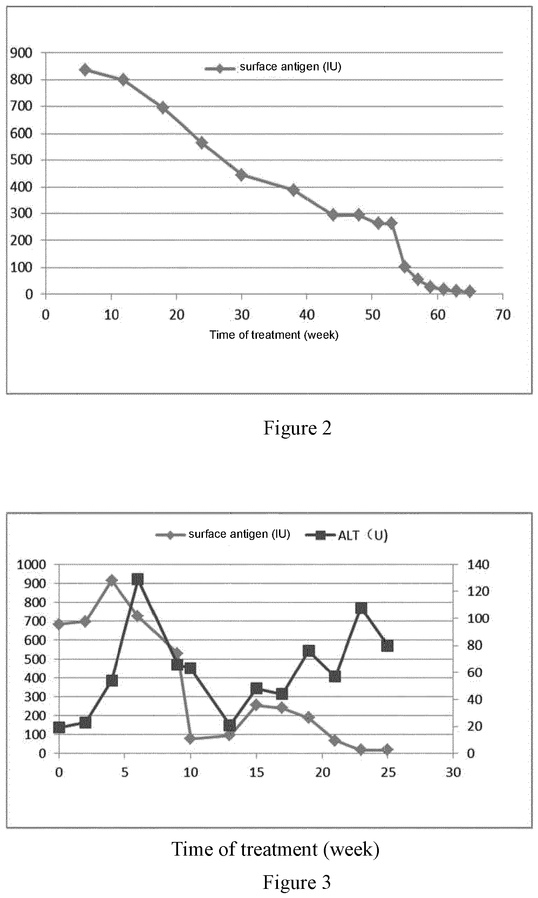

Specifically, as shown in FIG. 2, Pegberon was administered to the patient in Week 0 to Week 48, and the nucleotide analogue ETV was administrated since Week 49. Pegberon administration was suspended in Week 49 to Week 52, and resumed from Week 53 to Week 61. Pegberon was discontinued for more than 4 weeks since Week 62. It may be seen from the figure that the decline in surface antigens during Week 53 to Week 65, during which the administration is implemented for 8 weeks and suspended for 4 weeks, is significantly faster than that in Week 36 to Week 48. It is shown that the recovery of immune clearance capability may have achieved in the 5 weeks during which the administration is discontinued.

In the treatment of another patient with chronic hepatitis B infection, treatment with Pegberon combined with ETV was implemented for 8 weeks, and then administration of Pegberon was discontinued for 5 weeks while the use of ETV was continued, and then Pegberon was used for 5 weeks and discontinued for 2 weeks.

Administration of Pegberon: 90 micrograms per week. The nucleotide analogue ETV was administrated in 0.5 mg per day.

As shown in FIG. 3, during the use of interferon, it was observed after the first period of 4 weeks that the surface antigen increased with the increase of transaminase, indicating that the interferon may activate the relevant cell clearance mechanism. It was observed, after discontination for 5 weeks and resumed administration for 2 weeks, the process of increased transaminase and increased surface antigens. It is suggested that after treatment with interferon, discontinuation for a certain period of time and resumed administration may repeatedly start the immune clearance. Relevant immunosuppression may be released by the discontinuation. It was speculated that elevated transaminases means that the infected cells were cleared by immunity. While the destroyed liver cells released related metabolic enzymes, the surface antigens were temporarily increased along with the release of surface antigens from the destroyed cells.

In the treatment of another patient with chronic hepatitis B infection, Pegberon was administered in 180 micrograms per week, the nucleotide analog ETV was administered in 0.5 mg per day. Starting from Week 4, Pegberon was administered in combination with ETV for 24 weeks. In Week 28- 40, Pegberon was discontined, and solely ETV was administered. In Week 44-68, Pegberon was administered in combination with ETV. In Week 68-76, Pegberon was discontined, and solely ETV was administered. In Week 76-80, Pegberon was administered in combination with ETV.

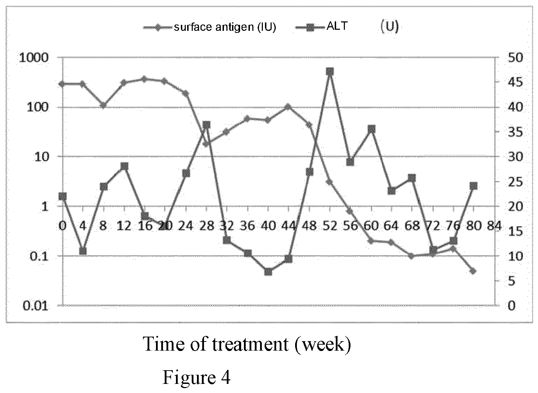

As shown in FIG. 4, during the use of interferon, it was observed in the first period of 24 weeks that the surface antigen increased before decreasing along with the increase of transaminase, indicating that interferon may activate the relevant cell clearance mechanism. In the treatment of Week 76-80, the surface antigens were less than 0.05, such that the surface antigens of hepatitis B were lower than the detection line, thereby eliminating the surface antigens. It is suggested that after treatment with interferon, discontinuation for a certain period of time and resumed administration may repeatedly start the immune clearance.

The above results suggested that through the cycled administration, the immune clearance and consumption of immune cells may be repeated during the interferon administration, and during the discontinuation phase, the nucleotide analog was used to prevent viral reinfection, while allowing partial immune recovery and eliminating immune suppression, whereby eventually eliminating the liver cells infected with hepatitis B virus, such that the surface antigens in the serum of the patient with chronic hepatitis B turned negative to achieve clinical cure.

### Example 3. Preparation of recombinant mouse interferon α4 (mIFN-α4)

Interferons are also widely used in cancer treatment, but the effect needs to be improved. In order to study whether the continuous administration of interferon actually causes partial immune suppression and exhaustion of immune cells, which leads to poor subsequent treatment effects, the efficacy of interferon intermittent administration and continuous administration was compared in mice.

A large number of studies on anti-tumor effects were carried out in nude mice (lack of normal thymus) as the host. As the immune status of nude mice is weaker than that of normal mice, it is difficult to reflect the immune response under the normal state. In the anti-tumor model based on normal mice, the effect of interferon on the immune system may be partially realized, which reflects the effect of the anti-tumor therapy of administering the interferon. From the perspective of function realization, interferon should rely on the host's immune system to play its full anti-viral and anti-tumor effects. Because interferon has strong species specificity, when normal mice were used as the research subjects in the examples, the use of murine interferon or derivatives thereof may better reflect its physiological effects and implementation effects. In the research model using the mice of the present invention, PEGylated recombinant mouse interferon α4 (PEG-mIFNα4) was used as a representative of interferon therapeutic drugs and their derivatives.

According to the amino acid sequence of mIFN-α4 (GenBank NP 034634), the cDNA sequence of mIFN-α4 was optimized and designed according to the preferred codons of Pichia pastoris, and GenScript Biotechnology Co., Ltd. was entrusted to synthesize the cDNA. The cDNA encoding mIFN-α4 was recombined into pPIC9K plasmid, transformed into TOP10 competent cells, plated on LB solid medium, and cultured overnight at 37°C. A single clone was picked to inoculate the LB liquid medium, which was then cultured overnight at 37°C. The plasmid was extracted, and double digestion was performed with XhoI and NotI. Positive clones were identified by nucleic acid electrophoresis, and further confirmed by nucleic acid sequencing. The positive clone plasmid was digested and linearized with SalI, electrotransformed into Pichia pastoris GS115, plated on the RD plate, and cultured at 28-30°C for 3 days. The positive transformants were picked to inoculate YPD liquid medium, which was cultured overnight at 28-30°C, and transfered to BMMY medium at a final concentration of OD600nm of about 1, and incubated at 28-30°C for about 24 hours. Methanol was added until the final concentration of methanol of about 1 %, and then further cultured at 28-30 °C for about 24 hours. The culture medium was centrifuged to collect the supernatant, and the expression of mIFN-α4 was detected by SDS-PAGE electrophoresis. According to the results of SDS-PAGE electrophoresis, the engineered strains with higher expression and stable expression were selected for subsequent fermentation in a fermenter.

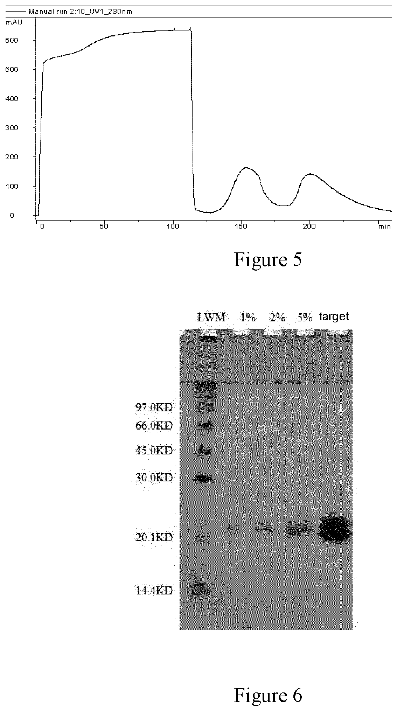

The fermenter was 30L. Refer to the "Pichia Fermentation Process Guidelines" for fermentation culture and methanol induction, the induction time was about 30h. The fermentation supernatant was collected by centrifugation, and concentrated by ultrafiltration for 3~5 times in 5kD hollow fiber membrane tube, and the buffer system was replaced with 20mM phosphate buffer-20mM arginine hydrochloride-50mM sodium chloride buffer solution (pH6.5). SP Sepharose Fast Flow chromatography column (GE Healthcare, column bed Φ38mm×160mm) was then loaded, 20mM phosphate buffer -20mM arginine hydrochloride (pH6.5) (solution A) was then used to wash about 3 column volumes. The solution A and 20mM phosphate buffer -20mM arginine hydrochloride - 1M sodium chloride solution (pH6.5) (solution B) were used to perform gradient elution. mIFN-α4 target samples were collected, and sample for non-reducing SDS-PAGE (with the separation gel concentration of 14%, silver staining) were taken. The elution profile is shown in FIG. 5, and the electrophoresis result is shown in FIG. 6.

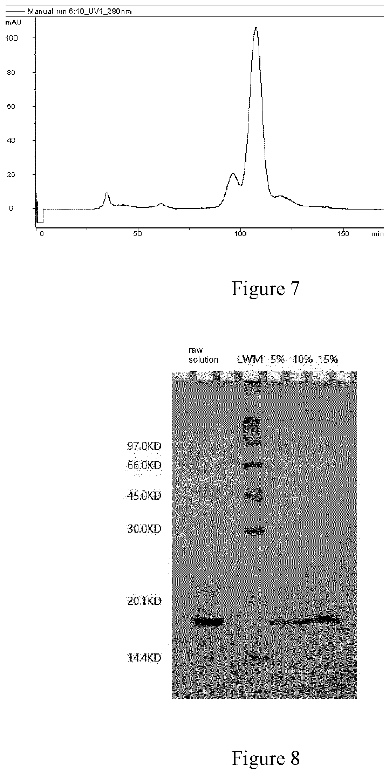

mIFN-α4 SP Sepharose Fast Flow purification sample was concentrated by ultrafiltration with 5K ultrafiltration membrane package and replaced with 20mM phosphate buffer-50mM arginine hydrochloride-lOmM methionine-20mM sodium chloride (pH 7.0), and then its concentration was adjusted to about 1.0mg/ml. Glycosidase was added at a mass ratio of mIFN-α4 protein to enzyme of about 20:1, and digestion was performed at 25°C for about 20 hours to remove glycosyl groups. The digested sample was diluted by about 6 times with 5mM boric acid buffer-10mM arginine hydrochloride (pH9.0), and the sample was loaded on Q Sepharose Fast Flow chromatography column (GE Healthcare, column bed Φ50mm×154mm). 20mM boric acid buffer -20mM arginine hydrochloride-lOmM methionine (pH 9.0) (solution C) was used to wash about 3 column volumes. The solution C and 20mM boric acid buffer -20mM arginine hydrochloride-lOmM formazan thionine-0.3M sodium chloride (pH 9.0) (solution D) were used for gradient elution. The target deglycosylated mIFN-α4 sample was collected, and the pH was adjusted to about pH 5.0 with 10% acetic acid. 5K ultrafiltration membrane was used to concentrate by ultrafiltration and the buffer was replaced with 5mM acetic acid/sodium acetate buffer-50mM arginine hydrochloride-lOOmM sodium chloride (pH 5.0). The resulting sample was the stock solution of deglycosylated mIFN-α4. Samples were taken to be sent for inspection, and the remaining samples were frozen at -70°C for later use. The elution profile is shown in FIG. 7, and the SDS-PAGE electrophoresis result is shown in FIG. 8.

The bacterial endotoxin content of the deglycosylated mIFN-α4 stock solution was determined by the Limulus reagent method as lower than 60 EU/mg. specific activity was determined as 5.4×10⁸U/mg by using the commercial mIFN-α4 (R&D, catalog number 12115-1) as the standard, and by using the mouse fibroblast/encephalomyocarditis virus (L929/EMCV) cytopathic inhibition method.

### Example 4. Preparation of PEGed recombinant mouse interferon (PEG-mIFN-α4)

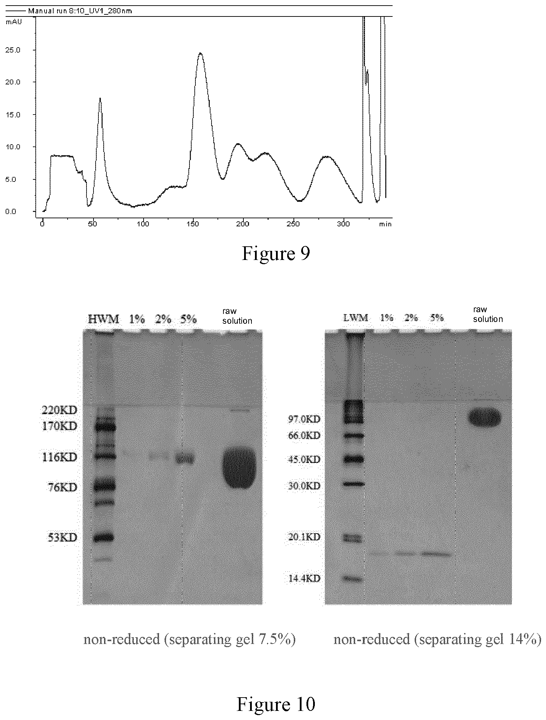

The deglycosylated mIFN-α4 stock solution was replaced through ultrafiltration with buffer of 5 mM acetic acid/sodium acetate buffer-50mM sodium chloride (pH5.0) with 5kD ultrafiltration membrane package. About 333ml sample (with deglycosylatedmIFNα4 content of about 500mg) was taken, added with about 22ml of 0.8M boric acid/sodium hydroxide buffer (pH 9.4), and stirred well. YPEG-NHS was added based on the mass ratio of protein to 40kD Y-type polyethylene glycol succinimide ester (YPEG-NHS) of about 1:8, which was then stirred quickly, and reacted at room temperature for 10 minutes. Then about 20ml of 0.2M methionine was added to stop the reaction, and the pH was adjusted to 5.0 with 10% acetic acid. 550ml pure water was then added, and then 600ml 20mM acetic acid/sodium acetate buffer-20mM arginine hydrochloride-lOmM methionine (pH5.1) (solution E), mixed well. After that, it was loaded on the SP Sepharose Fast Flow chromatography column (GE Healthcare, column bed Φ50mm×194mm), and the solution E was used to wash about 5 column volumes. After that, the solution E and 20mM acetic acid/sodium acetate buffer-20mM arginine hydrochloride-lOmM methionine-600mM Sodium chloride (pH5.1) (solution F) were used for gradient elution, and PEG-mIFN-α4 target samples were collected. The buffer was replaced through ultrafiltration by using the 5kD ultrafiltration membrane package to 20mM phosphate buffer-123mM sodium chloride (pH 6.5). and then concentrated appropriately, added with 0.5% Tween80 to the final concentration of Tween80 being about 0.005%. The resulting sample was the PEG-mIFN-α4 stock solution (PEG-mIFN-α4). Samples were taken and then sent for inspection, the remaining samples were frozen and stored at -70°C for later use. The elution profile is shown in FIG. 9, and the SDS-PAGE electrophoresis result is shown in FIG. 10.

The bacterial endotoxin content of the PEG-mIFN-α4 stock solution was determined by the Limulus reagent method as lower than 15 EU/mg. Specific activity was determined as 6.1 × 10⁶U/mg by using the commercial mIFN-α4 (R&D, catalog number 12115-1) as the standard, and by using the mouse fibroblast/encephalomyocarditis virus (L929/EMCV) cytopathic inhibition method.

### Example 5. Pharmacokinetic study of subcutaneous injection of PEG-mIFN-α4 in healthy BALB/c mice

In this example, BALB/c mice were purchased from Beijing Vital River Laboratory Animal Technology Co., Ltd. The blood concentration of PEG-mIFN-α4 was detected by double-antibody sandwich ELISA. The test kit of the Mouse IFN alpha Platinum ELISA kit (Cat. No. BMS6027/ BMS6027TEN, Thermo) was used, but the reporter antibody was replaced with the anti-PEG antibody 3.3 biotin (Institute of Biomedical Sciences, Academia Sinica (IBMS), Taiwan) to avoid interference of endogenous mIFN-α on the measurement result.

6-8 weeks old, SPF grade BALB/c mice (N=60, with fifty-fifty of male and female mice) were injected with a single injection of 1 µg/mouse PEG-mIFN-α4 subcutaneously on the back of the neck. Plasma samples were collected before the injection (0h) and after the injection at 15h, 24h, 36h, 48h, 72h, 96h, 120h, 168h, 216h, 264h, 312h, to determine the blood concentration. Phoenix WinNonlin 6.4 software was used to calculate the pharmacokinetic parameters.

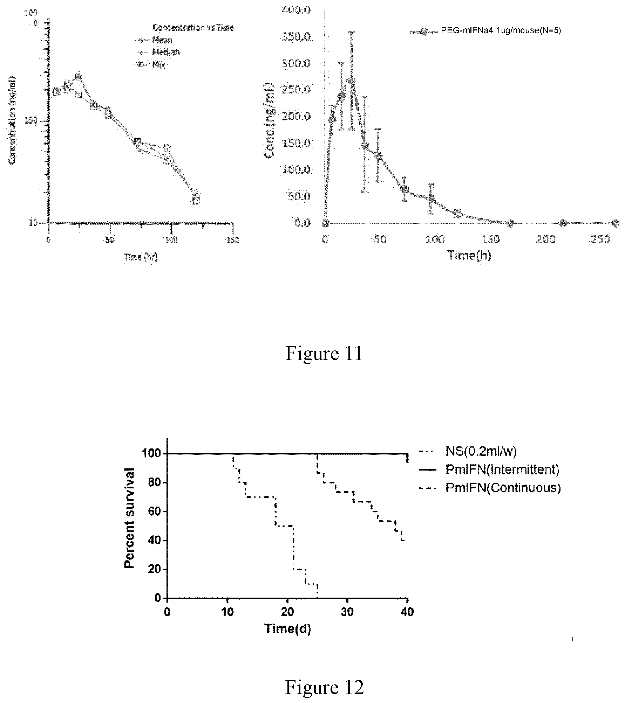

The results of blood concentration determination are shown in Table 1, the blood concentration-administration time curve is shown in FIG. 11, and the results of pharmacokinetic parameters are shown in Table 2. With a single injection of 1µg/mouse of PEG-mIFN-α4 subcutaneously on the back of the neck of BALB/c mice, plasma concentration peak time (Tₘₐₓ) was 24h, peak concentration time (Tₘₐₓ) was 268ng/ml, and elimination half-life (T_{1/2}) was 28.3h.

**Table 1. Results of blood concentration determination of BALB/c mice with a single subcutaneous injection of 1µg/mouse of PEG-mIFN-α4 (N=5, ng/ml)**

| **Collected at** | **Individual blood concentration (ng/ml)** | | | | | **Mean (ng/ml)** | **SD** |
|---|---|---|---|---|---|---|---|
| | 1 | 2 | 3 | 4 | 5 | | |
| 0h | 0 | 0 | 0 | 0 | 0 | 0.0 | 0.0 |
| 6h | 218.1 | 165.9 | 167.8 | 220.8 | 200.6 | 194.6 | 26.5 |
| 15h | 282.7 | 326.1 | 194.3 | 203.8 | 183.2 | 238.0 | 62.9 |
| 24h | 341.5 | 290.8 | 362.8 | 185.3 | 159.7 | 268.0 | 91.5 |
| 36h | 204.8 | 263.1 | 145.4 | 60.6 | 61.4 | 147.1 | 88.9 |
| 48h | 205.8 | 128.1 | 133.3 | 79 | 91.4 | 127.5 | 49.5 |
| 72h | 95.5 | 73.6 | 40.4 | 52.9 | 54.6 | 63.4 | 21.5 |
| 96h | 41.2 | 52.6 | 87.5 | 20.6 | 22.2 | 44.8 | 27.4 |
| 120h | 22.6 | 26.1 | 19.1 | 11.5 | 10.6 | 18.0 | 6.8 |
| 168h | BLQ | BLQ | BLQ | BLQ | BLQ | BLQ | / |
| 216h | BLQ | BLQ | BLQ | BLQ | BLQ | BLQ | / |
| 264h | BLQ | BLQ | BLQ | BLQ | BLQ | BLQ | / |
| 312h | BLQ | BLQ | BLQ | BLQ | BLQ | BLQ | / |

| | | | | | | | |
|---|---|---|---|---|---|---|---|
| Note: In the pharmacokinetic analysis, for samples with blood concentration lower than the quantification lower limit, the sample before the peak blood concentration was counted as 0, and the sample after the peak was expressed as BLQ. | | | | | | | |

**Table 2. Summary of pharmacokinetic parameters of a single subcutaneous injection of 1µg/mouse of PEG-mIFN-α4 in BALB/c mice**

| **Parameter** | **Unit** | PEG-mIFNα4 |
|---|---|---|
| T_{1/2} | hr | **28.3** |
| Tₘₐₓ | hr | 24.0 |
| Cmax | ng/ml | 268.0 |
| Tₗₐₛₜ | hr | 120.0 |
| AUCₗₐₛₜ | hr^{∗}ng/ml | 13289.3 |
| AUCINF_{_obs} | hr^{∗}ng/ml | 14023.2 |
| Vz_F_{_obs} | ml | 2.91 |
| Cl_F_{_obs} | ml/hr | 0.07 |

### Example 6. Comparative Study on the treatment efficacy of PEG-mIFN-α4 intermittent administration (administered once every 48 hours, continuously administered for 3 times, suspended for 144 hours; 4 consecutive rounds), and continuous administration (administered once every 48 hours, continuously administered for 21 times) for transplanted liver cancer H₂₂ in mice.

In this example, BALB/c mice were purchased from Beijing Vital River Laboratory Animal Technology Co., Ltd., and the mouse liver cancer H₂₂ cell line was purchased from the China Center for Type Culture Collection (CCTCC).

6-8 weeks old, 18-22g SPF grade BALB/c mice were subcutaneously inoculated with H₂₂ cells (0.5×10⁶ cells/mL, 0.2 ml/mouse) under the armpit of the right forelimb. Randomized grouping was made on the day of tumor inoculation. Experimental groups included PEG-mIFN-α4 continuous administration group (N=15, 7 males and 8 females), PEG-mIFN-α4 intermittent administration group (N=15, 7 males and 8 females) and normal saline control group (N=10, 5 females and 5 males) and so on.

PEG-mIFN-α4 was administered by subcutaneous injection at the back of the neck, at a dosage of 1 microgram per animal. The PEG-mIFN-α4 intermittent administration group was administered once every 48 hours for 3 times, then suspended for 144 hours, repeated for 4 rounds. The PEG-mIFN-α4 continuous administration group was administered once every 48 hours, continuously administered for 21 times. The normal saline control group was given an equal volume of normal saline.

All groups were subjected to survival analysis to compare differences between groups. SAS 9.4 and office2010 software were used for statistical analysis, and the statistical tests were all two-sided tests.

The survival curve is shown in FIG. 12, and the statistical comparison results are shown in Table 3. On Day 40 after tumor inoculation, the median survival time of the normal saline control group was 19.5 days, and that of the PEG-mIFN-α4 continuous administration group was 38 days. The survival rate of the PEG-mIFN-α4 intermittent administration group was 93.3%, while the median survival time cannot be calculated yet. The survival curves of the PEG-mIFN-α4 continuous administration group and the intermittent administration group were significantly different from the normal saline control group. The difference between the PEG-mIFN-α4 continuous administration group and the intermittent administration group was also significant (P=0.0035). The survival period of the PEG-mIFN-α4 intermittent administration group was significantly longer than that of the continuous administration group. For the treatment of transplanted liver cancer H₂₂ in mice, intermittent administration of PEG-mIFN-α4 shows significantly better curative effect than continuous administration.

**Table 3. Survival analysis results of comparative experiment on the efficacy of continuous and intermittent administration of PEG-mIFN-α4 in the treatment of transplanted liver cancer H₂₂ in mice**

| **Group** | **Number of cases (Female + male)** | **Median survival period (Time after tumor inoculation, days)** | **Survival curve comparison** | |
|---|---|---|---|---|
| | | | **P value¹** | **P value²** |
| Normal saline control group | 5+5 | 19.5 | / | / |
| PEG-mIFN-α4 intermittent administration group | 7+8 | Survival rate during the observation period was 93.3%. | <0.0001 ^{∗} | / |
| PEG-mIFN-α4 continuous administration group | 7+8 | 38.0 | 0.0137^{∗} | 0.0035^{∗} |

| | | | | |
|---|---|---|---|---|
| Note: 1. Compared with the "normal saline control group". 2. Compared with "PEG-mIFN-α4 intermittent administration group". 3. ^{∗}: Statistically significant difference. | | | | |

### Example 7. Intermittent administration of interferon combined with gemcitabine to treat liver cancer H₂₂ in mice

The inventors studied the antitumor effect of intermittent administration of PEGylated recombinant mouse interferon α (PEG-mIFN-α4) combined with gemcitabine on mouse liver cancer H22, and explored the efficacy of combined administration of interferon and gemcitabine. The inventors surprisingly found that by intermittently administering PEG-mIFN-α4 and simultaneously administering gemcitabine, significantly better results may be obtained in the treatment of H22 tumors, compared to administering gemcitabine alone or intermittently administering PEG-mIFN-α4 alone.

In this example, BALB/c mice were purchased from Beijing Vital River Laboratory Animal Technology Co., Ltd., and the H22 mouse liver cancer cell strain was purchased from China Center for Type Culture Collection (CCTCC), and the manufacturer of the anticancer agent gemcitabine was Jiangsu Hansoh Pharmaceutical Group Co., Ltd.

120 6-8 weeks old, 18-22g SPF grade BALB/c mice were subcutaneously inoculated with hepatocellular carcinoma H22 tumor cells (1×10⁶ cells/mL, 0.2 ml/mouse) in the right forelimb axillary. Three days after the tumor was inoculated, they were randomly divided into several study groups including the normal saline control group, PEG-mIFN-α4 only group, gemcitabine only group, and PEG-mIFN-α4 combined with gemcitabine group. The administration was started on the day after grouping, once a week. The PEG-mIFN-α4 was injected subcutaneously on the back of the neck, and gemcitabine was injected intraperitoneally. The average tumor volume for each individual group was calculated based on the formula V=1/2ab² (a and b represent length and width (mm) respectively) by measuring the tumor diameter, and the anti-tumor effect was observed dynamically.

SAS 9.4, office2010 software were used for statistical analysis. Statistical tests were all two-sided tests.

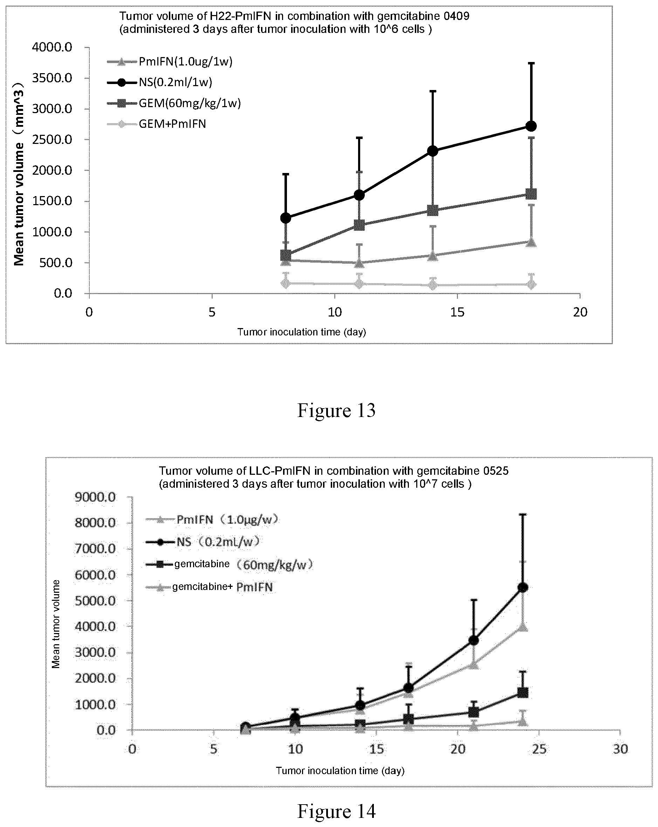

The changes of tumor volume in each group are shown in FIG. 13. After 8 days of tumor inoculation, the tumors of all mice in the normal saline control group were visible to the naked eye. 18 days after the tumor inoculation, the average tumor volume of the administration group was lower than that of the saline group, suggesting that gemcitabine, PEG-mIFN-α4, PEG-mIFN-α4 combined with gemcitabine all generated certain anti-tumor effects. The average tumor volume of the PEG-mIFN-α4 combined with gemcitabine group was significantly lower than that of the PEG-mIFN-α4 only group (P<0.0001) and the gemcitabine only group (P<0.0001).

The results showed that the anti-cancer activity of intermittent administration of peginterferon murine α combined with gemcitabine was significantly better than that of intermittent administration of peginterferon α 4 alone or gemcitabine alone.

**Table 4. Summary and comparison of the average tumor volume after administration to each group in Example 7**

| **Group** | **Number of cases (Female + male)** | **Mean tumor volume (Standard deviation)/mm^³** | **Inter-group comparison** | |
|---|---|---|---|---|
| | | | **P value¹** | P **value²** |
| PEG-mIFN-α4 (1.0µg/w) | 13+13 | 846.2 (592.0) | **<0.0001*** | **<0.0001*** |
| Normal saline (0.2mL/w) | 13+13 | 2722.0 (1023.2) | | |
| Gemcitabine (60mg/kg/w) | 13+13 | 1619.3 (911.2) | **0.0001*** | **<0.0001*** |
| Gemcitabine+PEG-mIFN-α4 | 13+13 | 148.6 (164.2) | | |

| | | | | |
|---|---|---|---|---|
| Note: P value¹ was compared with "normal saline (0.2ml/w)"; P value² was compared with "gemcitabine + PEG-mIFN-α4"; and ^{∗} refers to a statistical difference. | | | | |

### Example 8. Intermittent administration of interferon combined with gemcitabine to treat lung cancer LLC in mice

The inventors studied the antitumor effect of intermittent administration of PEG-mIFN-α4 combined with gemcitabine on mouse lung cancer LLC, and explored the efficacy of combined administration of interferon and gemcitabine. It was found through the experiment that by intermittently administering PEG-mIFN-α4 and simultaneously administering gemcitabine, significantly better results may also be obtained in the treatment of lung cancer LLC, compared to administering gemcitabine alone or intermittently administering PEG-mIFN-α4 alone.

In this example, C57BL/6N mice were purchased from Beijing Vital River Laboratory Animal Technology Co., Ltd., and the LLC mouse lung cancer cell line was purchased from Cell Resource Center, IBMS. The anticancer agent was gemcitabine (Jiangsu Hansoh Pharmaceutical Group Co., Ltd).

120 6-8 weeks old, 18-22g SPF grade C57BL6/N mice were subcutaneously inoculated with lung cancer LLC tumor cells (1×10⁷ cells/mL, 0.2ml/mouse) in the right forelimb axillary. 3 days after tumor inoculation, they were randomly divided into normal saline control group, gemcitabine only group, PEG-mIFN-α4 combined with gemcitabine group. The administration was started on the day after grouping, once a week. The PEG-mIFN-α4 was injected subcutaneously on the back of the neck, and gemcitabine was injected intraperitoneally. The average tumor volume for each individual group was calculated based on the formula V=1/2ab² (a and b represent length and width (mm) respectively) by measuring the tumor diameter, and the anti-tumor effect was observed dynamically. SAS 9.4, office2010 software were used for statistical analysis. Statistical tests were all two-sided tests.

The changes of tumor volume in each group are shown in FIG. 14. After 8 days of tumor inoculation, the tumors of all mice in the normal saline control group were visible to the naked eye. 24 days after the tumor inoculation, the average tumor-bearing volume of the administration group was lower than that of the saline group, suggesting that gemcitabine, PEG-mIFN-α4, and PEG-mIFN-α4 combined with gemcitabine all generated certain anti-tumor effects. The average tumor volume of the PEG-mIFN-α4 combined with gemcitabine group was significantly lower than that of the PEG-mIFN-α4 only group (P<0.0001) and the gemcitabine only group (P<0.0001).

The results showed that the anti-lung-cancer activity of intermittent administration of peginterferon murine α combined with gemcitabine was significantly better than that of intermittent administration of peginterferon α 4 alone or administration of gemcitabine alone.

**Table 5. Summary and comparison of the average tumor volume after administration to each group in Example 8**

| **Group** | **Number of cases (Female + male)** | **Mean tumor volume (Standard deviation)/mm^³** | **Inter-group comparison** | |
|---|---|---|---|---|
| | | | **P value¹** | **P value²** |
| PEG-mIFN-α4 (1.0µg/w) | 14+14 | 4000.8 (2513.7) | **0.0388** | **<0.0001*** |
| Normal saline (0.2mL/w) | 14+14 | 5511.6 (2817.1) | | |
| Gemcitabine (60mg/kg/w) | 14+14 | 1466.6 (791.5) | **<0.0001*** | **<0.0001*** |
| Gemcitabine+PEG-mIFN-α4 | 14+14 | 364.1 (387.8) | | |

| | | | | |
|---|---|---|---|---|
| Note: P value¹ was compared with "normal saline (0.2ml/w)"; P value² was compared with "gemcitabine + PEG-mIFN-α4"; and ^{∗} refers to a statistical difference. | | | | |

### Example 9. Intermittent administration of interferon combined with gemcitabine to treat colorectal cancer CT26 in mice

The inventors studied the antitumor effect of intermittent administration of PEG-mIFN-α4 combined with gemcitabine on mouse colorectal cancer CT26, and explored the efficacy of combined administration of interferon and gemcitabine. It was found through the experiment that by intermittently administering PEG-mIFN-α4 and simultaneously administering gemcitabine, significantly better results may also be obtained in the treatment of colorectal cancer CT26, compared to administering gemcitabine alone or intermittently administering PEG-mIFN-α4 alone. In this example, BALB/c mice were purchased from Beijing Vital River Laboratory Animal Technology Co., Ltd., and the CT26 mouse colorectal cancer cell line was purchased from Cell Resource Center, Shanghai Academy of Biological Sciences, Chinese Academy of Sciences. The anticancer agent was gemcitabine (Jiangsu Hansoh Pharmaceutical Group Co., Ltd). 120 6-8 weeks old, 18-22g SPF grade BALB/c mice were subcutaneously inoculated with colorectal cancer CT26 tumor cells (1×10⁶ cells/mL, 0.2 ml/mouse) in the right forelimb axillary. Three days after the tumor was inoculated, they were randomly divided into several study groups including the normal saline control group, PEG-mIFN-α4 only group, gemcitabine only group, and PEG-mIFN-α4 combined with gemcitabine group. The administration was started on the day after grouping, once a week. PEG-mIFNα was injected subcutaneously on the back of the neck, and gemcitabine was injected intraperitoneally. The average tumor volume for each individual group was calculated based on the formula V=1/2ab² (a and b represent length and width (mm) respectively) by measuring the tumor diameter, and the anti-tumor effect was observed dynamically.

SAS 9.4, office2010 software were used for statistical analysis. Statistical tests were all two-sided tests.

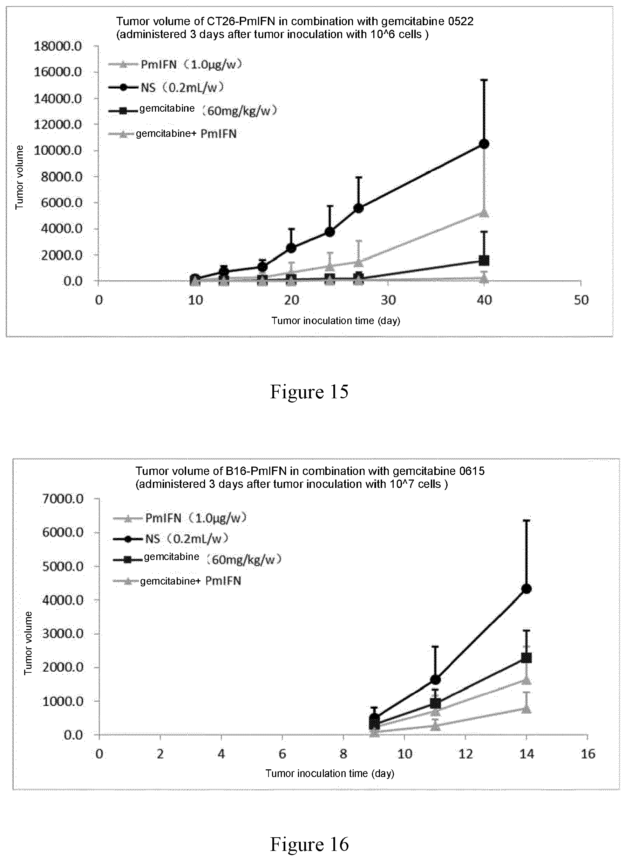

The changes of tumor volume in each group are shown in FIG. 15. After 8 days of tumor inoculation, the tumors of all mice in the normal saline control group were visible to the naked eye. By day 43, the average tumor-bearing volume of the administration group was significantly lower than that of the normal saline group (P<0.05), and the average tumor-bearing volume of the administration group was lower than that of the normal saline group, suggesting that gemcitabine, PEG-mIFN-α4, PEG-mIFN- α4 combined with gemcitabine have certain anti-tumor effects. The average tumor volume of the PEG-mIFN-α4 combined with gemcitabine group was significantly lower than that of the PEG-mIFN-α4 only group (P<0.0001) and the gemcitabine only group (P=0.0054).

The results showed that the anti-colorectal-cancer activity of intermittent administration of peginterferon murine α combined with gemcitabine was significantly better than that of intermittent administration of peginterferon α 4 alone or administration of gemcitabine alone.

**Table 6. Summary and comparison of the average tumor volume after administration to each group in Example 9**

| **Group** | **Number of cases (Female + male)** | **Mean tumor volume (Standard deviation)/mm^³** | **Inter-group comparison** | |
|---|---|---|---|---|
| | | | P **value¹** | **P value²** |
| PEG-mIFN-α4 (1.0µg/w) | 14+12 | 5283.7 (5030.5) | **0.0004*** | **<0.0001*** |
| Normal saline (0.2mL/w) | 14+12 | 10510.8 (4910.8) | | |
| Gemcitabine (60mg/kg/w) | 14+13 | 1558.2 (2225.2) | **<0.0001*** | **0.0054*** |
| Gemcitabine+PEG-mIFN-α4 | 14+13 | 238.9 (460.0) | | |

| | | | | |
|---|---|---|---|---|
| Note: P value¹ was compared with "normal saline (0.2ml/w)"; P value² was compared with "gemcitabine + PEG-mIFN-α4"; and ^{∗} refers to a statistical difference. | | | | |

### Example 10. Intermittent administration of interferon combined with gemcitabine to treat melanoma B16 in mice

The inventors studied the antitumor effect of intermittent administration of PEG-mIFN-α4 combined with gemcitabine on mouse melanoma B16, and explored the efficacy of combined administration of interferon and gemcitabine. It was found through the experiment that by intermittently administering PEG-mIFN-α4 and simultaneously administering gemcitabine, significantly better results may also be obtained in the treatment of melanoma B16, compared to administering gemcitabine alone or intermittently administering PEG-mIFN-α4 alone.

In this example, C57BL/6N mice were purchased from Beijing Vital River Laboratory Animal Technology Co., Ltd., and the B16 mouse melanoma cell line was from ATCC. The anticancer agent was gemcitabine (Jiangsu Hansoh Pharmaceutical Group Co., Ltd).

120 6-8 weeks old, 18-22g SPF grade C57BL/6N mice were subcutaneously inoculated with B16 tumor cells (1×10⁷ cells/mL, 0.2 ml/mouse) in the right forelimb axillary. Three days after the tumor was inoculated, they were randomly divided into several study groups including the normal saline control group, PEG-mIFN-α4 only group, gemcitabine only group, and PEG-mIFN-α4 combined with gemcitabine group. The administration was started on the day after grouping, once a week. The PEG-mIFN-α4 was injected subcutaneously on the back of the neck, and gemcitabine was injected intraperitoneally. The average tumor volume for each individual group was calculated based on the formula V=1/2ab² (a and b represent length and width (mm) respectively) by measuring the tumor diameter, and the anti-tumor effect was observed dynamically.

SAS 9.4, office2010 software were used for statistical analysis. Statistical tests were all two-sided tests.

The changes of tumor volume in each group are shown in FIG. 16. After 8 days of tumor inoculation, the tumors of all mice in the normal saline control group were visible to the naked eye. 14 days after the tumor inoculation, the average tumor-bearing volume of the administration group was lower than that of the saline group, suggesting that gemcitabine, PEG-mIFN-α4, and PEG-mIFN-α4 combined with gemcitabine all generated certain anti-melanoma effects. The average tumor volume of the PEG-mIFN-α4 combined with gemcitabine group was significantly lower than that of the PEG-mIFN-α4 only group (P=0.0002) and the gemcitabine only group (P<0.0001).

The results showed that the anti-melanoma activity of intermittent administration of peginterferon murine α combined with gemcitabine was significantly better than that of intermittent administration of peginterferon α 4 alone or administration of gemcitabine alone.

**Table 7. Summary and comparison of the average tumor volume after administration to each group in Example 10**

| **Group** | **Number of** c **cases (Female + male)** | **Mean tumor volume (Standard deviation)/mm^³** | **Inter-group comparison** | |
|---|---|---|---|---|
| | | | **P value¹** | **P value²** |
| PEG-mIFN-α4 (1.0µg/w) | 14+14 | 1639.7 (979.5) | **<0.0001*** | 0.0002^{∗} |
| Normal saline (0.2mL/w) | 14+14 | 4330.9 (2028.6) | | |
| Gemcitabine (60mg/kg/w) | 14+14 | 2279.9 (807.6) | **<0.0001*** | <0.0001^{∗} |
| Gemcitabine+PEG-mIFN-α4 | 14+14 | 804.1 (467.6) | | |

| | | | | |
|---|---|---|---|---|
| Note: P value¹ was compared with "normal saline (0.2ml/w)"; P value² was compared with "gemcitabine + PEG-mIFN-α4"; and ^{∗} refers to a statistical difference. | | | | |

### Example 11. Comparative study on the efficacy of intermittent administration of PEG-mIFN-α4 (administered once every 48 hours, for 3 times continuously, before discontinuation of 144 hours) in combination with the anticancer agent gemcitabine and administration of gemcitabine alone on transplanted liver cancer H₂₂ in mice.

In this example, BALB/c mice were purchased from Beijing Vital River Laboratory Animal Technology Co., Ltd., and the H₂₂ mouse liver cancer cell strain was purchased from China Center for Type Culture Collection (CCTCC), and the manufacturer of the anticancer agent gemcitabine was Jiangsu Hansoh Pharmaceutical Group Co., Ltd.

6-8 weeks old, 18-22g SPF grade BALB/c mice were subcutaneously inoculated with liver cancer H₂₂ tumor cells (1× 10⁶cells/mL, 0.2ml/mouse) in the right forelimb axillary. 3 days after tumor inoculation, they were randomly divided into groups, with 26 mice in each group (fifty-fifty female and male mice), including normal saline control group, gemcitabine group, and PEG-mIFN-α4 combined with gemcitabine group. After being divided into groups, the corresponding drugs or saline were administered. PEG-mIFN-α4 was administered subcutaneously on the back of the neck, in a dosage of 1 microgram per mouse. With respect to the intermittent administration, it was administered once every 48 hours, for three times continuously, before discontinuation of 144 hours. Gemcitabine was administered by intraperitoneal injection at a dose of 60 mg per kilogram of body weight each time, once a week. The normal saline control group was given an equal volume of normal saline. The PEG-mIFN-α4 intermittent administration combined with gemcitabine group was given gemcitabine during the first injection of PEG-mIFN-α4, and then administered with gemcitabine once a week thereafter.

All groups were subjected to survival analysis to compare differences between groups. SAS 9.4 and office2010 software were used for statistical analysis, and the statistical tests were all two-sided tests.

By day 41, the mortality rate of the normal saline control group was 100% (26/26), that of the gemcitabine group was 73.1% (19/26), and that of the PEG-mIFN-α4 intermittent administration combined with gemcitabine group was 3.8% (1 /26). The median survival time of the saline control group was 15.5 days, that of the gemcitabine group was 21.5 days, and that of the survival rate of the PEG-mIFN-α4 combined with gemcitabine intermittent administration group was 96.2%.

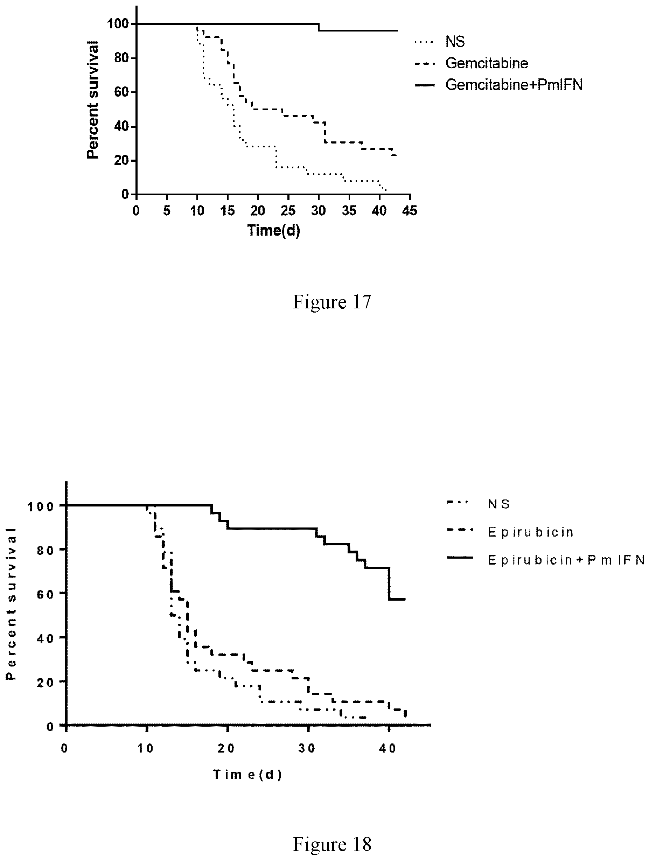

The survival curve is shown in FIG. 17, and the statistical comparison results are shown in Table 8. The survival curve of the PEG-mIFN-α4 intermittent administration combined with gemcitabine group was significantly different from that of the saline control group (P<0.0001), and also significantly different from the gemcitabine group (P<0.0001). For the treatment of transplanted liver cancer H₂₂ in mice, the intermittent administration of PEG-mIFN-α4 in combination with the positive drug gemcitabine showed better efficacy than the administrator of the positive control drug gemcitabine.

The results showed that the anticancer activity of intermittent administration of PEG-mIFN-α4 combined with gemcitabine was significantly better than that of gemcitabine alone.

**Table 8. Survival analysis results of comparative experiments on efficacy of intermittent administration of PEG-mIFN-α4 combined with gemcitabine in the treatment of transplanted liver cancer H₂₂ in mice**

| **Group** | **Number of cases (Female + male)** | **Median survival period (Time after tumor inoculation, days)** | **Survival curve comparison** | |
|---|---|---|---|---|
| | | | **P value¹** | **P value²** |
| Normal saline control group | 13+13 | 15.5 | / | / |
| Gemcitabine Group | 13+13 | 21.5 | 0.0279 | / |
| PEG-mIFN-α4 intermittent administration Combined with Gemcitabine Group | 13+13 | Survival rate during the observation period was 96.2%. | <0.0001 * | <0.0001^{∗} |

| | | | | |
|---|---|---|---|---|
| Note: 1. The P value was the result by comparing with the "normal saline control group"; 2. The P value was the result by comparing with "gemcitabine group"; 3. ^{∗} refers to a statistical difference. | | | | |

### Example 12. Study on the treatment of transplanted liver cancer H₂₂ in mice by intermittent administration of interferon and anticancer agent epirubicin

The anticancer agent used in this example is the antitumor antibiotic epirubicin among chemotherapeutics, and the effect of intermittent administration of interferon combined with anticancer agent epirubicin in the treatment of transplanted liver cancer H₂₂ in mice was investigated. In this example, BALB/c mice were purchased from Beijing Vital River Laboratory Animal Technology Co., Ltd., and the mouse liver cancer H₂₂ cell line was purchased from the China Center for Type Culture Collection (CCTCC). The manufacturer of epirubicin was Beijing Union Pharmaceutical Factory.

6-8 weeks old, 18-22g SPF grade BALB/c mice were subcutaneously inoculated with liver cancer H₂₂ tumor cells (1× 10⁶ cells/mL, 0.2ml/mouse) in the right forelimb axillary. 3 days after tumor inoculation, they were randomly divided into groups, with 28 mice in each group (fifty-fifty female and male mice), including normal saline control group, epirubicin only group, and PEG-mIFN-α4 combined with epirubicin group.

The administration was started on the day after grouping, once a week. PEG-mIFN-α4 was injected subcutaneously on the back of the neck, and epirubicin was injected intraperitoneally, in a dose of 3.5 mg/kg each time. The normal saline control group was administered an equal volume of normal saline.

All groups were subjected to survival curve analysis, and the median survival time was compared. SAS 9.4 and office2010 software were used for statistical analysis. Statistical tests were all two-sided tests.

The survival curve is shown in FIG. 18, and the median survival time is shown in Table 9. The median survival time of the saline group was 13.5 days, the median survival time of the epirubicin group was 15 days, and the survival rate of the PEG-mIFN-α4 combined with epirubicin group during the observation period was 57.1%. The survival curve of PEG-mIFN-α4 combined with epirubicin group was significantly different from that of normal saline group (P<0.0001). It was showed that the survival time of tumor-bearing mice may be significantly prolonged (P<0.0001) by intermittent administration of PEG-mIFN-α4 combined with epirubicin, compared with administration of epirubicin alone.

This example suggests that in the treatment of transplanted liver cancer H₂₂ in mice, the effect of intermittent administration of PEG-mIFN-α4 in combination with epirubicin was significantly better than that of epirubicin alone.

**Table 9. Survival analysis results of Example 12**

| **Group** | **Number of cases (Female**+ male) | **Median survival period (Time after tumor inoculation, days)** | **Survival curve comparison** | |
|---|---|---|---|---|
| | | | **P** value¹ | **P value²** |
| Normal saline (0.2mL/w) | 14+14 | 13.5 | / | |
| Epirubicin (3.5mg/kg/w) | 14+14 | 15.0 | 0.4598 | |
| Epirubicin+PEG-mIFN-α4 | 14+14 | Survival rate during the observation period was 57.1%. | <0.000 1^{∗} | <0.0001^{∗} |

| | | | | |
|---|---|---|---|---|
| Note: P value¹ was compared with "normal saline (0.2ml/w)"; P value² was compared with "epirubicin (3.5mg/kg/w)"; and ^{∗} refers to a statistical difference. | | | | |

### Example 13. Study on the treatment of transplanted liver cancer H₂₂ in mice by intermittent administration of interferon in combination with anticancer agent oxaliplatin

The anticancer agent used in this example is the chemotherapeutic agent oxaliplatin, and the effect of intermittent administration of interferon combined with anticancer agent oxaliplatin in the treatment of transplanted liver cancer H₂₂ in mice was investigated.

In this example, BALB/c mice were purchased from Beijing Vital River Laboratory Animal Technology Co., Ltd., and the H₂₂ mouse liver cancer cell line was purchased from the China Center for Type Culture Collection (CCTCC). The manufacturer of oxaliplatin was Qilu Pharmaceutical (Hainan) Co., Ltd.

6-8 weeks old, 18-22g SPF grade BALB/c mice were subcutaneously inoculated with liver cancer H₂₂ tumor cells (1× 10⁶ cells/mL, 0.2ml/mouse) in the right forelimb axillary. 3 days after tumor inoculation, they were randomly divided into groups, with 28 mice in each group (fifty-fifty female and male mice), including normal saline control group, oxaliplatin only group, and PEG-mIFN-α4 combined with oxaliplatin group.

The administration was started on the day after grouping, once a week. PEG-mIFN-α4 was injected subcutaneously on the back of the neck, and oxaliplatin was injected intraperitoneally, in a dose of 10 mg/kg each time. The normal saline control group was administered an equal volume of normal saline.

All groups were subjected to survival curve analysis, and the median survival time was compared. SAS 9.4 and office2010 software were used for statistical analysis. Statistical tests were all two-sided tests.

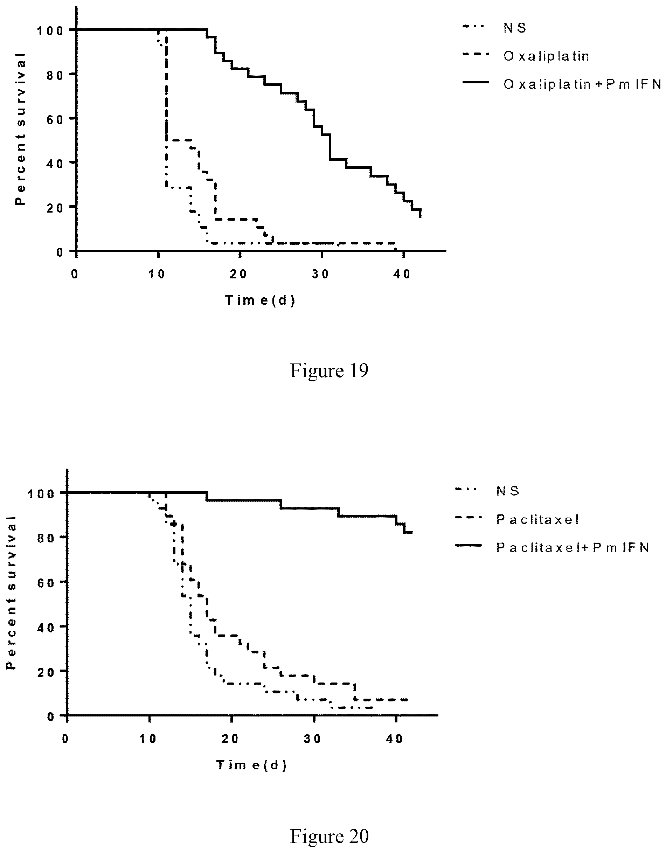

The survival curve is shown in FIG. 19, and the median survival time is shown in Table 10. The median survival time of the normal saline group was 11 days, the median survival time of the oxaliplatin group was 12.5 days, and the median survival time of the PEG-mIFN-α4 combined with oxaliplatin group was 31 days. The survival curve of the PEG-mIFN-α4 combined with oxaliplatin group was significantly different from that of the normal saline group (P=0.0001), and also significantly different from the oxaliplatin group alone (P=0.0014). Intermittent administration of PEG -mIFN-α4 combined with oxaliplatin may significantly prolong the survival time of tumor-bearing mice.

This example suggests that in the treatment of transplanted liver cancer H₂₂ in mice, the effect of intermittent administration of PEG-mIFN-α4 in combination with oxaliplatin was significantly better than that of oxaliplatin alone.

**Table 10. Survival analysis results of Example 13**

| **Group** | **Number of cases (Female + male)** | **Median survival period (Time after tumor inoculation, days)** | **Survival curve comparison** | |
|---|---|---|---|---|
| | | | **P value¹** | **P value²** |
| Normal saline (0.2mL/w) | 14+14 | 11.0 | / | / |
| Oxaliplatin (10mg/kg/w) | 14+14 | 12.5 | 0.5112 | / |
| Oxaliplatin+PEG-mIFN-α4 | 14+14 | 31.0 | **0.0001*** | **0.0014*** |

| | | | | |
|---|---|---|---|---|
| Note: P value¹ was compared with "normal saline (0.2ml/w)"; P value² was compared with "oxaliplatin (10mg/kg/w)"; and ^{∗} refers to a statistical difference. | | | | |

### Example 14. Study on the treatment of transplanted liver cancer H₂₂ in mice by intermittent administration of interferon and anticancer agent paclitaxel

The anticancer agent used in this example is the chemotherapeutic agent paclitaxel, and the effect of intermittent administration of interferon combined with anticancer agent paclitaxel in the treatment of transplanted liver cancer H₂₂ in mice was investigated.

In this example, BALB/c mice were purchased from Beijing Vital River Laboratory Animal Technology Co., Ltd., and the H₂₂ mouse liver cancer cell line was purchased from the China Center for Type Culture Collection (CCTCC). The manufacturer of paclitaxel was Qilu Pharmaceutical (Hainan) Co., Ltd.

6-8 weeks old, 18-22g SPF grade BALB/c mice were subcutaneously inoculated with liver cancer H₂₂ tumor cells (1× 10⁶ cells/mL, 0.2ml/mouse) in the right forelimb axillary. 3 days after tumor inoculation, they were randomly divided into groups, with 28 mice in each group (fifty-fifty female and male mice), including normal saline control group, paclitaxel only group, and PEG-mIFN-α4 combined with paclitaxel group.

The administration was started on the day after grouping, once a week. PEG-mIFN-α4 was injected subcutaneously on the back of the neck, in a dose of 1 mg/mouse each time, and oxaliplatin was injected intraperitoneally, in a dose of 10 mg/kg each time.

All groups were subjected to survival curve analysis, and the median survival time was compared.

SAS 9.4 and office2010 software were used for statistical analysis. Statistical tests were all two-sided tests.

The survival curve is shown in FIG. 20, and the median survival time is shown in Table 11. The median survival time of the normal saline group was 15 days, and that of the paclitaxel group was 17 days. During the observation period, the survival rate of the PEG-mIFN-α4 combined with paclitaxel group was 82.1%. The survival curve of the PEG-mIFN-α4 combined with paclitaxel group was significantly different from that of the normal saline group (P=0.0001), and also significantly different from the paclitaxel group alone (P<0.0001). Intermittent administration of PEG -mIFN-α4 combined with paclitaxel may significantly prolong the survival time of tumor-bearing mice.

This example suggests that in the treatment of transplanted liver cancer H₂₂ in mice, the effect of intermittent administration of PEG-mIFN-α4 in combination with paclitaxel was significantly better than that of paclitaxel alone.

**Table 11. Survival analysis results of Example 14**

| **Group** | **Number of cases (Female + male)** | **Median survival period (Time after tumor inoculation, days)** | **Survival curve comparison** | |
|---|---|---|---|---|
| | | | **P value¹** | **P value²** |
| Normal saline (0.2mL/w) | 14+14 | 15.0 | / | / |
| Paclitaxel (10mg/kg/w) | 14+14 | 17.0 | 0.3147 | / |
| Paclitaxel + PEG-mIFN-α4 | 14+14 | Survival rate during the observation period was 82.1%. | <0.000 1* | **<0.0001*** |

| | | | | |
|---|---|---|---|---|
| Note: P value¹ was compared with "normal saline (0.2ml/w)"; P value² was compared with "paclitaxel (10mg/kg/w)"; and ^{∗} refers to a statistical difference. | | | | |

The above results of the present invention suggest that by intermittently administering PEG-mIFN-α4 in combination with a chemotherapeutic agent such as gemcitabine, paclitaxel, oxaliplatin, epirubicin, etc., it is possible to obtain a significantly better cancer treatment effect than administering the chemotherapeutic agent alone.

Similar results in the treatment of hepatitis B and cancer suggest that continuous administration of interferon for long time will cause immunosuppression and depletion of immune cells, which is difficult to recover. The efficacy of interferon depends on the immune system. Therefore, an interferon-based therapy may achieve good therapeutic effects such as high virus clearance or tumor suppression in the early stage of treatment when immune cells are still sufficient. However, in the later stage of treatment, due to depletion of immune cells or immunosuppression caused by long-term administration of interferon, the therapeutic effect may be substantially reduced, and high efficacy may not be maitained even if interferon is continued to be administered. This problem may be avoided by intermittent administration of interferons. For example, after administering interferon for a period of time to achieve a certain effect and before partial immune suppression and immune cell exhaustion, the administration of interferon can be suspended for a period of time so that immune cells can recover as soon as possible, and then re-administration of interferon can still achieve better therapeutic efficacy. By intermittently administering the interferon therapeutic agent in reasonable combination with another drug, better therapeutic effects may be achieved.

### Sequences and description

>SEQ ID NO: 1 is the amino acid sequence of interferon α 2a (e.g., Roferon-A or Pegasys) 
CDLPQ THSLG SRRTL MLLAQ MRKIS LFSCL KDRHD FGFPQ EEFGN QFQKA ETIPV
LHEMI QQIFN LFSTK DSSAA WDETL LDKFY TELYQ QLNDL EACVI QGVGV TEPLM
KEDSI LAVRK YFQRI TLYLK EKKYS PCAWE VVRAE IMRSF SLSTN LQESL RSKE
>SEQ ID NO: 2 is the amino acid sequence of interferon α 2b (e.g., INTRONA or PEG-INTRON or Pegberon) 


。
>SEQ ID NO: 3 is the amino acid sequence of a recombinant integrated interferon (e.g., Infergen) 
MCDLPQTHSL GNRRALILLA QMRRISPFSC LKDRHDFGFP QEEFDGNQFQ
KAQAISVLHE MIQQTFNLFS TKDSSAAWDE SLLEKFYTEL YQQLNDLEAC
VIQEVGVEET PLMNVDSILA VKKYFQRITL YLTEKKYSPC AWEVVRAEIM RSFSLSTNLQ
ERLRRKE。
>SEQ ID NO: 4 is the amino acid sequence of integrated interferon α-2 (e.g., PEGINFER) 
GSGGGCDLPQTHSLGNRRALILLAQMRRISPFSCLKDRHDFGFPQEEFDGNQFQKAQAISV
LHEMIQQTFNLFSTKDSSAAWDESLLEKFYTELYQQLNDLEACVIQEVGVEETPLMNVDSI
LAVRKYFQRITLYLTEKKYSPCAWEVVRAEIMR SFSLSTNLQERLRRKD
>SEQ ID NO: 5 is the amino acid sequence of interferon λ 
MGPVP TSKPT TTGKG CHIGR FKSLS PQELA SFKKA RDALE ESLKL KNWSC SSPVF
PGNWD LRLLQ VRERP VALEA ELALT LKVLE AAAGP ALEDV LDQPL HTLHH ILSQL
QACIQ PQPTA GPRPR GRLHH WLHRL QEAPK KESAG CLEAS VTFNL FRLLT RDLKY
VADGN LCLRT STHPE ST
>SEQ ID NO: 6 is the amino acid sequence of peginterferon murine α 4

CDLPHTYNLGNKRALTVLEEMRRLPPLSCLKDRKDFGFPLEKVDNQQIQKAQAILVLRDL
TQQILNLFTSKDLSATWNATLLDSFCNDLHQQLNDLKACVMQEPPLTQEDSLLAVRTYFH
RITVYLRKKKHSLCAWEVIRAEVWRALSSSTNLLARLSEEKE

### References:

[1] Gisslinger H, et. al. Ropeginterferon alfa-2b, a novel IFNα-2b, induces high response rates with low toxicity in patients with polycythemia vera. Blood. 2015 Oct 8;126(15):1762-9. doi: 10.1182/blood-2015-04-637280. Epub 2015 Aug 10.
[2] Gane EJ, et. al. The oral toll-like receptor-7 agonist GS-9620 in patients with chronic hepatitis B virus infection. J Hepatol. 2015 Aug;63(2):320-8. doi: 10.1016/j.jhep.2015.02.037. Epub 2015 Feb 27.
[3] Janssen HLA, et. al. Safety, efficacy and pharmacodynamics of vesatolimod (GS-9620) in virally suppressed patients with chronic hepatitis B. J Hepatol. 2018 Mar;68(3):431-440. doi: 10.1016/j.jhep.2017.10.027. Epub 2017 Dec 11.
[4] Agarwal K, et. al. Safety and efficacy of vesatolimod (GS-9620) in patients with chronic hepatitis B who are not currently on antiviral treatment. Journal of Viral Hepatitis [2018 Aug, 25(11):1331-1340.
[5] Lopatin U, et. al. Safety, pharmacokinetics and pharmacodynamics of GS-9620, an oral Toll-like receptor 7 agonist. Antivir Ther. 2013;18(3):409-18. doi: 10.3851/IMP2548. Epub 2013 Feb 15.
[6] Edward J. Gane, et. al. TLR7 agonist RO7020531 triggers immune activation after multiple doses in chronic hepatitis B patients Reported by Jules Levin. AASLD 2018 Nov 9-13 SF
[7] Ebrahim M, et. al. Are RIG-1 and MDA5 Expressions Associated with Chronic HBV Infection? Viral Immunol. 2015 Nov;28(9):504-8. doi: 10.1089/vim.2015.0056. Epub 2015 Oct 20.
[8] Ma Z, et. al. Contribution of Toll-like receptors to the control of hepatitis B virus infection by initiating antiviral innate responses and promoting specific adaptive immune responses. Cell Mol Immunol. 2015 May;12(3):273-82. doi: 10.1038/cmi.2014.112. Epub 2014 Nov 24.
[9] Jones M, et. al. SB 9200, a novel agonist of innate immunity, shows potent antiviral activity against resistant HCV variants. J Med Virol. 2017 Sep;89(9):1620-1628. doi: 10.1002/jmv.24809. Epub 2017 May 23.
[10] Xu CL, et. al. Upregulation of toll-like receptor 4 on T cells in PBMCs is associated with disease aggravation of HBV-related acute-on-chronic liver failure. J Huazhong Univ Sci Technolog Med Sci. 2015 Dec;35(6):910-915. doi: 10.1007/s11596-015-1527-x. Epub 2015 Dec 16.
[11] Kato H, et. al. RIG-I-Like Receptors and Type I Interferonopathies. J Interferon Cytokine Res. 2017 May;37(5):207-213. doi: 10.1089/jir.2016.0095.
[12] Li L, et. al. Anti-HBV response to toll-like receptor 7 agonist GS-9620 is associated with intrahepatic aggregates of T cells and B cells. J Hepatol. 2018 May;68(5):912-921. doi: 10.1016/j.jhep.2017.12.008. Epub 2017 Dec 14.
[13] Harrington, et. al. LBA15Preliminary results of the first-in-human (FIH) study of MK-1454, an agonist of stimulator of interferon genes (STING), as monotherapy or in combination with pembrolizumab (pembro) in patients with advanced solid tumors or lymphomas. Annals of Oncology.2018 OCT.29(8):712-712.Meeting Abstract.
[14] Cemerski, Saso, et. al. Preclinical characterization of a novel STING agonist, MK-1454. JOURNAL FOR IMMUNOTHERAPY OF CANCER. 2017 NOV.5(2):16.Meeting Abstract.
[15] Ramanjulu JM, et. al. Design of amidobenzimidazole STING receptor agonists with systemic activity. Nature. 2018 Dec;564(7736):439-443. doi: 10.1038/s41586-018-0705-y. Epub 2018 Nov 7.
[16] Safety and Efficacy of MIW815 (ADU-S100) +/- Ipilimumab in Patients With Advanced/Metastatic Solid Tumors or Lymphomas. https://www.aduro.com/file.cfm/13/docs/SITC18_MIW815X2101_SITC%20Poster(e_version)_0 8Nov2018.pdf
[17]Lara PN Jr, et. al. Randomized phase III placebo-controlled trial of carboplatin and paclitaxel with or without the vascular disrupting agent vadimezan (ASA404) in advanced non-small-cell lung cancer. J Clin Oncol. 2011 Aug 1;29(22):2965-71. doi: 10.1200/JCO.2011.35.0660. Epub 2011 Jun 27.
[18] Yuen, M. F, et. al. Dose response and safety of the daily, oral RIG-I agonist Inarigivir (SB 9200) in treatment naive patients with chronic hepatitis B: results from the 25mg and 50mg cohorts in the ACHIEVE trial. JOURNAL OF HEPATOLOGY. 2018 APR.68(1): S509-S510.DOI: 10.1016/S0168-8278(18)31267-4
[19] Ma Z, et. al. Interaction between Hepatitis B Virus and Toll-Like Receptors: Current Status and Potential Therapeutic Use for Chronic Hepatitis B. Vaccines (Basel). 2018 Jan 16;6(1). pii: E6. doi: 10.3390/vaccines6010006.
[20] Walsh, R., et. al. Effects of SB9200 (Inarigivir) therapy on immune responses in patients with chronic hepatitis B.JOURNAL OF HEPATOLOGY. 2018 APR.68(1): S89-S89. DOI: 10.1016/S0168-8278(18)30396-9
[21] Niu C, et. al. Toll-like receptor 7 agonist GS-9620 induces prolonged inhibition of HBV via a type I interferon-dependent mechanism. J Hepatol. 2018 May;68(5):922-931. doi: 10.1016/j.jhep.2017.12.007. Epub 2017 Dec 13.
[22] Yuen, MF, et. al. Ascending dose cohort study of inarigivir - A novel RIG I agonist in chronic HBV patients: Final results of the ACHIEVE trial. JOURNAL OF HEPATOLOGY. 2019 APR.70(1): E47-E48.DOI: 10.1016/S0618-8278(19)30084-2
[23] Bourquin C, et. al. Harnessing the immune system to fight cancer with Toll-like receptor and RIG-I-like receptor agonists. Pharmacol Res. 2019 Mar 2. pii: S1043-6618(18)32066-8. doi: 10.1016/j.phrs.2019.03.001.
[24] Zeng Y, et. al. Toll-like receptors, long non-coding RNA NEAT1, and RIG-I expression are associated with HBeAg-positive chronic hepatitis B patients in the active phase. J Clin Lab Anal. 2019 Jun;33(5):e22886. doi: 10.1002/jcla.22886. Epub 2019 Mar 29.
[25] Wang Jiawen, et al. Research progress of Toll-like receptors and their agonists. Advances in Physiological Science, 2018, Vol. 49, No. 4, 289-292.
[26] Tao Zeyu, et al. Research progress of drugs for the treatment of chronic hepatitis B virus infection. Foreign Medicine and Antibiotics Volume, July 2018, Vol. 39, No. 4: 308-319.
[27] Xu Qun, et al. Research progress of immunomodulators targeting Toll-like receptors. Advances in Pharmacy, 2016, 40(1): 42-55.
[28] Liu Qiuming, et al. New Advances in Anti-HBV Drug Research, Journal of Virology. September 2016, Vol. 32, Issue 5: 650-658.
[29] Mu Na, et al. Expression levels of pattern recognition receptors TLR3, RIG-I and MDA5 in peripheral blood of patients with chronic hepatitis B. Chinese Journal of Immunology. 2016-05:715-719.
[30] Heinz Gisslinger, et. al. Ropeginterferon alfa-2b, a novel IFNα-2b, induces high response rates with low toxicity in patients with polycythemia vera.Blood. 2015 Oct 8; 126(15): 1762-1769.Prepublished online 2015 Aug 10.
[31] Monkarsh SP, et. al. Positional isomers of monopegylated interferon alpha-2a: isolation, characterization, and biological activity. Anal Biochem. 1997 May 1;247(2):434-40.
[32] Grace M, et. al. Structural and biologic characterization of pegylated recombinant IFN-alpha2b. J Interferon Cytokine Res. 2001 Dec;21(12):1103-15.
[33] Foser S, et. al. Isolation, structural characterization, and antiviral activity of positional isomers of monopegylated interferon alpha-2a (PEGASYS). Protein Expr Purif. 2003 Jul;30(1):78-87.
[34] Nanogen Pharmaceutical biotechbology.PEG-INTERFERON LAMBDA 1 CONJUGATES.WO2013/028233 A1. 28.02.2013.
[35] Nanogen Pharmaceutical biotechbology.PEG-INTERFERON LAMBDA 1 CONJUGATES.US 8454947 B1.Jun.4.2013. https://worldwide.espacenet.com/searchResults?submitted=true&locale=en_EP&DB=EPODOC& ST=advanced&TI=&AB=peginterferon+lambda&PN=&AP=&PR=&PD=&PA=&IN=&CPC=&IC=
[36] Wang Junzhi. Biotechnology drug research and development and quality control. 2nd edition. Science Press, 2007: 540.
[37] Interferon alpha-4 precursor [Mus musculus].https://www.ncbi.nlm.nih.gov/protein/NP_034634.
[38] Pichia Fermentation Process Guidelines, Thermo Fisher Scientific Inc.https://www.thermofisher.com/document-connect/document-connect.html?url=https% 3A%2F%2Fassets.thermofisher.com%2FTFS-Assets%2FLSG%2Fmanuals%2 Fpichiaferm_prot.pdf&title=UGljaGlhIEZlcm1lbnRhdGlvbiBHdWlkZWxpbmVz.

## Claims

1. A method for treating a disease in a subject, comprising intermittently administering an interferon-based therapeutic agent to the subject for a plurality of consecutive treatment courses.

2. The method according to claim 1, wherein the interferon-based therapeutic agent comprises an interferon or a mutant or derivative thereof, or comprises a nucleic acid molecule encoding an interferon or a mutant or a derivative thereof, or comprises a substance promoting the generation of an endogenous interferon.

3. The method according to claim 1 or 2, wherein the interferon is a Type I, Type II or Type III interferon, such as interferon α, interferon β, interferon γ or interferon λ, preferably interferon α.

4. The method according to any one of claims 1 to 3, wherein the interferon-based therapeutic agent comprises interferon α 2a, interferon α 2b, interferon α 1b, interferon λ, or a mutant or derivative thereof.

5. The method according to any one of claims 1 to 4, wherein the interferon or the mutant or derivative thereof is PEGylated.

6. The method according to any one of claims 1 to 5, wherein the interferon-based therapeutic agent is selected from the group consisting of P1101, Pegberon, Pegasys, Pegintron, Infergen, Novaferon, INTRONA, Roferon-A, Hapgen, PEGINFER and Peginterferon λ.

7. The method according to any one of claims 1 to 3, wherein the interferon-based therapeutic agent comprises an agonist of the TLRs, RLRs, and STINGs signaling pathways.

8. The method according to claim 7, wherein the interferon-based therapeutic agent is selected from the group consisting of GS-9620, GS-9688, RO7020531, RO6864018, TQ-A3334, JNJ-4964, SB9200, MIW815, DMXAA, MK-1454, and diABZI.

9. The method according to any one of claims 1-8, wherein in the consecutive treatment course, the interferon-based therapeutic agent is administered such that during substantially the entire course, the concentration of neopterin in the subject is higher than the concentration of neopterin before the first administration, for example approximately 110%, approximately 120%, approximately 130%, approximately 140%, approximately 150%, approximately 200%, approximately 250% or higher of the neopterin concentration before the first administration.

10. The method according to any one of claims 1-9, wherein the duration of the consecutive treatment course is the time period from the first administration to the last administration, plus about 5 in vivo half-lives of the therapeutic agent.

11. The method according to any one of claims 1-10, wherein the duration of each of the plurality of consecutive treatment courses is from about 1 week to about 24 weeks, preferably from about 1 week to about 12 weeks, further preferably from about 1 week to about 8 weeks, and yet further preferably about 2 weeks to about 6 weeks.

12. The method according to any one of claims 1-11, wherein the duration of each of the consecutive treatment courses is about 1 week to about 12 weeks, and the interval between the consecutive treatment courses is about 1 week to about 12 weeks.

13. The method according to any one of claims 1-12, wherein the interval between the consecutive treatment courses is from about 1 week to about 24 weeks, preferably from about 1 week to about 12 weeks, further preferably from about 1 week to about 8 weeks, and yet further preferably about 2 weeks to about 6 weeks.

14. The method according to any one of claims 1-13, wherein the duration of each of the consecutive treatment courses is about 1 week to about 8 weeks, and the interval between the consecutive treatment courses is about 1 week to about 8 weeks.

15. The method according to any one of claims 1-14, wherein the duration of each of the consecutive treatment courses is about 2 week to about 6 weeks, and the interval between the consecutive treatment courses is about 2 week to about 6 weeks.

16. The method according to any one of claims 1-15, wherein the interferon-based therapeutic agent is administered for 2-25 or more consecutive treatment courses.

17. The method according to any one of claims 1-16, wherein the durations of the plurality of consecutive treatment courses are substantially the same.

18. The method according to any one of claims 1-17, wherein the intervals between the consecutive treatment courses are substantially the same.

19. The method according to any one of claims 1-18, wherein the disease is a viral infection, for example, a viral infection selected from hepatitis B virus infection, hepatitis C virus infection, hepatitis D virus infection, HIV infection, EBV infection, HPV infection, CMV infection and herpes virus infection, preferably a hepatitis B virus infection.

20. The method according to any one of claims 1-18, wherein the disease is a cancer, for example, a cancer selected from leukemia (such as acute lymphocytic leukemia (ALL), acute myeloid leukemia (AML), chronic myeloid leukemia (CML), chronic lymphocytic leukemia, polycapillary leukemia), liver cancer, lung cancer, colorectal cancer, skin cancer, stomach cancer, breast cancer, prostate cancer, non-Hodgkin's lymphoma, melanoma, multiple myeloma, laryngeal papilloma, follicular lymphoma, AIDS-related Kaposi's sarcoma and renal cell carcinoma, preferably liver cancer, lung cancer, breast cancer, colorectal cancer or melanoma.

21. The method according to any one of claims 1-20, further comprising administering an additional agent to the subject.

22. The method according to claim 21, wherein the administration of the interferon-based therapeutic agent does not overlap with the administration of the additional agent.

23. The method according to claim 22, wherein the additional agent is administered between the plurality of consecutive treatment courses.

24. The method according to claim 21, wherein the administration of the interferon-based therapeutic agent overlaps with the administration of the additional agent.

25. The method according to claim 24, wherein the additional agent is administered during and between plurality of consecutive treatment courses.

26. The method according to any one of claims 21-25, wherein the additional agent is administered according to its conventional scheme.

27. The method according to any one of claims 21-26, wherein the additional agent is an antiviral agent.

28. The method according to claim 27, wherein the antiviral agent is a penetration and uncoating inhibitor such as amantadine, rimantadine, enfuvirtide, maraviro; DNA polymerase inhibitor such as acyclovir, ganciclovir, valacyclovir, famciclovir, foscarnet; a nucleoside reverse transcriptase inhibitor such as lamivudine, zidovudine, emtricitabine, Tenofovir, adefovir dipivoxil, TAF, entecavir, a reverse transcriptase inhibitor such as efavirenz, nevirapine; a protein inhibitor such as saquinavir; a neuraminidase inhibitor such as Oseltamivir, zanamivir; a broad-spectrum antiviral drug such as ribavirin.

29. The method according to any one of claims 21-28, wherein the interferon-based therapeutic agent is Pegberon and the antiviral agent is entecavir.

30. The method according to any one of claims 21-29, wherein the method is used for treatment of hepatitis B virus infection; the interferon-based therapeutic agent is Pegberon; the duration of each of the consecutive treatment courses is about 5 weeks to about 24 weeks, and the interval between the consecutive treatment courses is about 2 weeks to 8 weeks; and the antiviral agent is entecavir which is administered daily.

31. The method according to any one of claims 21-26, wherein the additional agent is an anticancer agent.

32. The method according to claim 31, wherein the anticancer agent is
i) a chemotherapeutic agent, such as an alkylating agent an alkylating agent: Nimustine, Carmustine, Lomustine, Cyclophosphamide, Ifosfamide, glyciphosphoramide, semustine; an antimetabolite: deoxyfluoguanosine, doxifluguanidine, 5-fluorouracil, mercaptopurine, thioguanine, cytarabine, fluguanosine, tegafur, Gemcitabine, carmofur, hydroxyurea, methotrexate, UFT, Ancitabine, capecitabine; an anti-tumor antibiotic: actinomycin D, doxorubicin, daunorubicin, Epirubicin, mitomycin, pelomycin, pingyangmycin, pirarubicin; a chemotherapeutic anti-tumor animal and plant ingredient: irinotecan, harringtonine, hydroxycamptothecin, Vinorelbine, paclitaxel, albumin paclitaxel, taxotere, topotecan, vincristine, vindesine, Vindesine, vinblastine, teniposide, etoposide, elemene; such as anti-tumor drug hormones: Atamestane, Anastrozole, Aminoglutethimide, Letrozole, Formestane, Metasterone, Tamoxifen; a chemotherapeutic miscellaneous agent: asparaginase, carboplatin, cisplatin, Dacarbazine, Oxaliplatin, Loxadine, Eloxatin, Mitoxantrone, or Procarbazine; or
ii) an immune checkpoint inhibitor such as an inhibitor of PD-1, PD-L1, CTLA4, for example, an antibody selected from: Nivolumab, Pembrolizumab, Atezolizumab, Durvalumab, Avelumab; or
iii) a small molecule targeting drug, such as imatinib, gefitinib, bortezomib, erlotinib, sorafenib, lenalidomide, Sunitinib, dasatinib, nilotinib, lapatinib, pazopanib, everolimus, vandetanib, crizotinib, verofinib, ruxolitinib, axitinib, vismodegib, carfilzomib, regorafenib, bosutinib, tofacitinib, carbotinib, panatinib, pomalidomide, trametinib, dabrafenib, Afatinib, Icotinib, Ibrutinib, Ceritinib, Idelaris, Apatinib, Pabuccilib, Levatinib, Axitinib, Icotinib , Apatinib, sonidegib, cobimetinib, osimertinib, alectinib, ixazomib; or
iv) a tumor-associated antigen-specific antibody such as Rituxan, Herceptin;
preferably, the anticancer agent is selected from oxaliplatin, epirubicin, paclitaxel and gemcitabine, and more preferably is gemcitabine.


Sequence listing

<110>  XIAMEN AMOYTOP BIOTECH CO., LTD.
       BIOSTEED GENE TRANSFORMATION TECH. Co., LTD.

<120>  INTERFERON-BASED DISEASE TREATMENT METHOD

<130>  AF1150 EP

<140>  EP 20 844 714.4
<141>  2020-07-22

<150>  201910660749.1
<151>  2019-07-22

<160>  6

<170>  PatentIn version 3.5

<210>  1
<211>  164
<212>  PRT
<213>  Artificial Sequence

<220>
<223>  ARTIFICIAL SEQUENCE

<400>  1

Cys Asp Leu Pro Gln Thr His Ser Leu Gly Ser Arg Arg Thr Leu Met
1               5                   10                  15

Leu Leu Ala Gln Met Arg Lys Ile Ser Leu Phe Ser Cys Leu Lys Asp
            20                  25                  30

Arg His Asp Phe Gly Phe Pro Gln Glu Glu Phe Gly Asn Gln Phe Gln
        35                  40                  45

Lys Ala Glu Thr Ile Pro Val Leu His Glu Met Ile Gln Gln Ile Phe
    50                  55                  60

Asn Leu Phe Ser Thr Lys Asp Ser Ser Ala Ala Trp Asp Glu Thr Leu
65                  70                  75                  80

Leu Asp Lys Phe Tyr Thr Glu Leu Tyr Gln Gln Leu Asn Asp Leu Glu
                85                  90                  95

Ala Cys Val Ile Gln Gly Val Gly Val Thr Glu Pro Leu Met Lys Glu
            100                 105                 110

Asp Ser Ile Leu Ala Val Arg Lys Tyr Phe Gln Arg Ile Thr Leu Tyr
        115                 120                 125

Leu Lys Glu Lys Lys Tyr Ser Pro Cys Ala Trp Glu Val Val Arg Ala
    130                 135                 140

Glu Ile Met Arg Ser Phe Ser Leu Ser Thr Asn Leu Gln Glu Ser Leu
145                 150                 155                 160

Arg Ser Lys Glu

<210>  2
<211>  165
<212>  PRT
<213>  ARTIFICIAL SEQUENCE

<220>
<223>  ARTIFICIAL SEQUENCE

<400>  2

<210>  3
<211>  167
<212>  PRT
<213>  ARTIFICIAL SEQUENCE

<220>
<223>  ARTIFICIAL SEQUENCE

<400>  3

Met Cys Asp Leu Pro Gln Thr His Ser Leu Gly Asn Arg Arg Ala Leu
1               5                   10                  15

Ile Leu Leu Ala Gln Met Arg Arg Ile Ser Pro Phe Ser Cys Leu Lys
            20                  25                  30

Asp Arg His Asp Phe Gly Phe Pro Gln Glu Glu Phe Asp Gly Asn Gln
        35                  40                  45

Phe Gln Lys Ala Gln Ala Ile Ser Val Leu His Glu Met Ile Gln Gln
    50                  55                  60

Thr Phe Asn Leu Phe Ser Thr Lys Asp Ser Ser Ala Ala Trp Asp Glu
65                  70                  75                  80

Ser Leu Leu Glu Lys Phe Tyr Thr Glu Leu Tyr Gln Gln Leu Asn Asp
                85                  90                  95

Leu Glu Ala Cys Val Ile Gln Glu Val Gly Val Glu Glu Thr Pro Leu
            100                 105                 110

Met Asn Val Asp Ser Ile Leu Ala Val Lys Lys Tyr Phe Gln Arg Ile
        115                 120                 125

Thr Leu Tyr Leu Thr Glu Lys Lys Tyr Ser Pro Cys Ala Trp Glu Val
    130                 135                 140

Val Arg Ala Glu Ile Met Arg Ser Phe Ser Leu Ser Thr Asn Leu Gln
145                 150                 155                 160

Glu Arg Leu Arg Arg Lys Glu
                165


<210>  4
<211>  171
<212>  PRT
<213>  Artificial Sequence

<220>
<223>  ARTIFICIAL SEQUENCE

<400>  4

Gly Ser Gly Gly Gly Cys Asp Leu Pro Gln Thr His Ser Leu Gly Asn
1               5                   10                  15

Arg Arg Ala Leu Ile Leu Leu Ala Gln Met Arg Arg Ile Ser Pro Phe
            20                  25                  30

Ser Cys Leu Lys Asp Arg His Asp Phe Gly Phe Pro Gln Glu Glu Phe
        35                  40                  45

Asp Gly Asn Gln Phe Gln Lys Ala Gln Ala Ile Ser Val Leu His Glu
    50                  55                  60

Met Ile Gln Gln Thr Phe Asn Leu Phe Ser Thr Lys Asp Ser Ser Ala
65                  70                  75                  80

Ala Trp Asp Glu Ser Leu Leu Glu Lys Phe Tyr Thr Glu Leu Tyr Gln
                85                  90                  95

Gln Leu Asn Asp Leu Glu Ala Cys Val Ile Gln Glu Val Gly Val Glu
            100                 105                 110

Glu Thr Pro Leu Met Asn Val Asp Ser Ile Leu Ala Val Arg Lys Tyr
        115                 120                 125

Phe Gln Arg Ile Thr Leu Tyr Leu Thr Glu Lys Lys Tyr Ser Pro Cys
    130                 135                 140

Ala Trp Glu Val Val Arg Ala Glu Ile Met Arg Ser Phe Ser Leu Ser
145                 150                 155                 160

Thr Asn Leu Gln Glu Arg Leu Arg Arg Lys Asp
                165                 170


<210>  5
<211>  182
<212>  PRT
<213>  ARTIFICIAL SEQUENCE

<220>
<223>  ARTIFICIAL SEQUENCE

<400>  5

Met Gly Pro Val Pro Thr Ser Lys Pro Thr Thr Thr Gly Lys Gly Cys
1               5                   10                  15

His Ile Gly Arg Phe Lys Ser Leu Ser Pro Gln Glu Leu Ala Ser Phe
            20                  25                  30

Lys Lys Ala Arg Asp Ala Leu Glu Glu Ser Leu Lys Leu Lys Asn Trp
        35                  40                  45

Ser Cys Ser Ser Pro Val Phe Pro Gly Asn Trp Asp Leu Arg Leu Leu
    50                  55                  60

Gln Val Arg Glu Arg Pro Val Ala Leu Glu Ala Glu Leu Ala Leu Thr
65                  70                  75                  80

Leu Lys Val Leu Glu Ala Ala Ala Gly Pro Ala Leu Glu Asp Val Leu
                85                  90                  95

Asp Gln Pro Leu His Thr Leu His His Ile Leu Ser Gln Leu Gln Ala
            100                 105                 110

Cys Ile Gln Pro Gln Pro Thr Ala Gly Pro Arg Pro Arg Gly Arg Leu
        115                 120                 125

His His Trp Leu His Arg Leu Gln Glu Ala Pro Lys Lys Glu Ser Ala
    130                 135                 140

Gly Cys Leu Glu Ala Ser Val Thr Phe Asn Leu Phe Arg Leu Leu Thr
145                 150                 155                 160

Arg Asp Leu Lys Tyr Val Ala Asp Gly Asn Leu Cys Leu Arg Thr Ser
                165                 170                 175

Thr His Pro Glu Ser Thr
            180

<210>  6
<211>  162
<212>  PRT
<213>  ARTIFICIAL SEQUENCE

<220>
<223>  ARTIFICIAL SEQUENCE

<400>  6

Cys Asp Leu Pro His Thr Tyr Asn Leu Gly Asn Lys Arg Ala Leu Thr
1               5                   10                  15

Val Leu Glu Glu Met Arg Arg Leu Pro Pro Leu Ser Cys Leu Lys Asp
            20                  25                  30

Arg Lys Asp Phe Gly Phe Pro Leu Glu Lys Val Asp Asn Gln Gln Ile
        35                  40                  45

Gln Lys Ala Gln Ala Ile Leu Val Leu Arg Asp Leu Thr Gln Gln Ile
    50                  55                  60

Leu Asn Leu Phe Thr Ser Lys Asp Leu Ser Ala Thr Trp Asn Ala Thr
65                  70                  75                  80

Leu Leu Asp Ser Phe Cys Asn Asp Leu His Gln Gln Leu Asn Asp Leu
                85                  90                  95

Lys Ala Cys Val Met Gln Glu Pro Pro Leu Thr Gln Glu Asp Ser Leu
            100                 105                 110

Leu Ala Val Arg Thr Tyr Phe His Arg Ile Thr Val Tyr Leu Arg Lys
        115                 120                 125

Lys Lys His Ser Leu Cys Ala Trp Glu Val Ile Arg Ala Glu Val Trp
    130                 135                 140

Arg Ala Leu Ser Ser Ser Thr Asn Leu Leu Ala Arg Leu Ser Glu Glu
145                 150                 155                 160

Lys Glu